# EUROPEAN PATENT SPECIFICATION

(11) **EP 4 583 828 B1**
(45) Date of publication and mention of the grant of the patent: **16.09.2026**
(21) Application number: 24775992.1
(22) Date of filing: 23.08.2024
(51) Int. Cl.: A61F 13/00, A61F 13/0246, A61K 9/70, A61L 15/00, A61L 15/44, A61B 17/04

(54) **WOUND CLOSURE SYSTEM HAVING SENSATE**
WUNDVERSCHLUSSSYSTEM MIT WAHRNEHMUNG
SYSTÈME DE FERMETURE DE PLAIE AYANT UN AGENT SENSORIEL

(30) Priority: 23.08.2023 US 202318454280
(43) Date of publication of application: 16.07.2025
(73) Proprietor: Cilag GmbH International, 6300 Zug (CH)
(72) Inventor: QUINTERO, Julian, Raritan, New Jersey 08869 (US); OU, Duan Li, Raritan, New Jersey 08869 (US); TANNHAUSER, Robert J., Raritan, New Jersey 08869 (US); KRIKSUNOV, Leo, Raritan, New Jersey 08869 (US); MIKSA, Davide, Raritan, New Jersey 08569 (US)
(74) Representative: Carpmaels & Ransford LLP
(86) International application number: PCT/IB2024/058216
(87) International publication number: WO 2025/041092

(56) References cited:
- US-A1- 2021 368 276
- US-A1- 2021 369 276
- US-A1- 2024 000 455

## Description

### BACKGROUND

A wound closure system (also referred to as a skin closure system) may be used at the conclusion of a surgical procedure on a patient to close a wound (e.g., a surgical incision) that was formed in the patient's skin for accessing a target anatomical structure. By way of example, wound closure systems may include components such as sutures, substrates, and/or liquid topical skin adhesives that are applied by a surgeon to approximate the edges of the wound and, in some cases, form a stable and protective layer over the wound that promotes efficient healing. In some instances, one or more components of the applied wound closure system may be absorbed by the patient during the healing process. Following healing of the wound, remaining components of the wound closure system may be removed from the skin by a surgeon, and/or they may automatically separate from the skin such that they may be discarded by the patient.

While various wound closure systems and associated components and methods have been made and used, it is believed that no one prior to the inventors has made or used the invention described in the appended claims.

### BRIEF DESCRIPTION OF THE DRAWINGS

The accompanying drawings, which are incorporated in and constitute a part of this specification, illustrate embodiments of the invention, and, together with the description given below, serve to explain the principles of the present invention.
FIG. 1 depicts a perspective view of an example of a wound closure system that includes a wound closure device, an adhesive applicator, and an adhesive spreader;
FIG. 2 depicts a disassembled perspective view of the wound closure device of FIG. 1, showing a mesh layer, a pressure sensitive adhesive layer, and a removable backing layer;
FIG. 3A depicts a perspective view of the wound closure device of FIG. 1 aligned longitudinally with a wound in the skin of a patient, showing a central section of the backing layer having been removed;
FIG. 3B depicts a perspective view of the wound closure device of FIG. 1 applied to the patient's skin over the wound to approximate the edges of the wound;
FIG. 3C depicts a perspective view of the wound closure device of FIG. 1 applied to the patient's skin over the wound, showing remaining sections of the backing layer having been removed so the wound closure device is fully adhered to the skin;
FIG. 3D depicts a perspective view of the wound closure device of FIG. 1 applied to the patient's skin over the wound, showing application of a liquid topical skin adhesive onto the mesh layer with the adhesive applicator;
FIG. 3E depicts a perspective view of the wound closure device of FIG. 1 applied to the patient's skin over the wound, showing the adhesive spreader positioned against the patient at the start of an adhesive spreading stroke for spreading the applied topical skin adhesive over and through the wound closure device;
FIG. 3F depicts a perspective view of the wound closure device of FIG. 1 applied to the patient's skin over the wound, showing the adhesive spreader during a subsequent portion of the adhesive spreading stroke;
FIG. 4 depicts a perspective view of an example of an alternative wound closure device having a sensate depot located on an underside thereof;
FIG. 5A depicts a perspective view of the wound closure device of FIG. 4 presented over a wound in the skin of a patient, showing an underside and sensate depot of the wound closure device;
FIG. 5B depicts a perspective view of the wound closure device of FIG. 4 applied to the patient's skin over the wound to approximate the edges of the wound, with the sensate depot sufficiently spaced away from the wound in the skin of the patient;
FIG. 5C depicts a perspective view of the wound closure device of FIG. 4 applied to the patient's skin over the wound, showing application of a liquid topical skin adhesive onto the mesh layer with the adhesive applicator;
FIG. 5D depicts a perspective view of the wound closure device of FIG. 4 applied to the patient's skin over the wound, showing the adhesive spreader positioned against the patient at the start of an adhesive spreading stroke for spreading the applied topical skin adhesive over and through the wound closure device;
FIG. 5E depicts a perspective view of the wound closure device of FIG. 4 applied to the patient's skin over the wound, the liquid topical skin adhesive spread onto and within the mesh layer;
FIG. 6 depicts a perspective view of an example of an alternative wound closure device having a sensate depot located on an underside thereof;
FIG. 7 depicts a perspective view of an example of an alternative wound closure device having a sensate depot located on an underside thereof;
FIG. 8 depicts a cross-sectional view of an alternative wound closure device having a sensate depot located on an upper side thereof;
FIG. 9 depicts a cross-sectional view of an alternative wound closure device having a sensate depot located flush with an underside thereof;
FIG. 10 depicts a cross-sectional view of an alternative wound closure device having a sensate depot extending downward from an underside thereof;
FIG. 11 depicts a cross-sectional view of an alternative wound closure device having a sensate depot extending both upwardly from an upper side of the wound closure device and downwardly from an underside thereof;
FIG. 12A depicts a cross-sectional view of an alternative wound closure device applied to the patient's skin over the wound, prior to application of a liquid topical skin adhesive;
FIG. 12B depicts a cross-sectional view of the wound closure device of FIG. 12A applied to the patient's skin over the wound, after application of a liquid topical skin adhesive, where a sensate depot of the wound closure device is in communication with skin of the patient, yet isolated from the wound of the patient;
FIG. 13A depicts a cross-sectional view of an alternative wound closure device applied to the patient's skin over the wound, prior to application of a liquid topical skin adhesive;
FIG. 13B depicts a cross-sectional view of the wound closure device of FIG. 13A applied to the patient's skin over the wound, after application of a liquid topical skin adhesive, where a sensate depot of the wound closure device is in communication with skin of the patient via a pressure sensitive adhesive, where the sensate depot is isolated from the wound of the patient;
FIG. 14A depicts a cross-sectional view of an alternative wound closure device applied to the patient's skin over the wound, prior to application of a liquid topical skin adhesive;
FIG. 14B depicts a cross-sectional view of the wound closure device of FIG. 14A applied to the patient's skin over the wound, after application of a liquid topical skin adhesive, where a pressure sensitive adhesive having a sensate depot is in communication with skin of the patient, where the sensate depot is isolated from the wound of the patient;
FIG. 15A depicts a cross-sectional view of an alternative wound closure device applied to the patient's skin over the wound, prior to application of a liquid topical skin adhesive;
FIG. 15B depicts a cross-sectional view of the wound closure device of FIG. 15A applied to the patient's skin over the wound, after application of a liquid topical skin adhesive, where a sensate depot of the wound closure device is in communication with skin of the patient, yet isolated from the wound of the patient;
FIG. 16A depicts a cross-sectional view of an alternative wound closure device applied to the patient's skin over the wound, prior to application of a liquid topical skin adhesive;
FIG. 16B depicts a cross-sectional view of the wound closure device of FIG. 16A applied to the patient's skin over the wound, after application of a liquid topical skin adhesive, where a sensate depot of the wound closure device is in communication with skin of the patient, yet isolated from the wound of the patient via a recess filled with cured topical skin adhesive;
FIG. 17A depicts a cross-sectional view of an alternative wound closure device applied to the patient's skin over the wound, prior to application of a liquid topical skin adhesive;
FIG. 17B depicts a cross-sectional view of the wound closure device of FIG. 17A applied to the patient's skin over the wound, after application of a liquid topical skin adhesive, where a sensate depot of the wound closure device is in communication with skin of the patient, yet isolated from the wound of the patient via a sponge filled with cured topical skin adhesive;
FIG. 18A depicts a cross-sectional view of an alternative wound closure device applied to the patient's skin over the wound after application of a liquid topical skin adhesive, where the wound closure device includes a plurality of rupturable sensate packets in communication with skin of the patient, yet isolated from the wound of the patient;
FIG. 18B depicts a cross-sectional view of the wound closure device of FIG. 18A applied to the patient's skin over the wound after application of a liquid topical skin adhesive, where the plurality of rupturable sensate packets of FIG. 18A are ruptured to release sensate toward the patient's skin;
FIG. 19A depicts a cross-sectional view of an alternative wound closure device applied to the patient's skin over the wound after application of a liquid topical skin adhesive, where the wound closure device includes a plurality of blister sensate packets in communication with skin of the patient, yet isolated from the wound of the patient; and
FIG. 19B depicts a cross-sectional view of the wound closure device of FIG. 19A applied to the patient's skin over the wound after application of a liquid topical skin adhesive, where the plurality of blister sensate packets of FIG. 19A are ruptured to release sensate toward the patient's skin.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "side," "upwardly," and "downwardly" also are used herein for reference to relative positions and directions. Such terms are used below with reference to views as illustrated for clarity and are not intended to limit the invention described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose described herein.

### I. Wound Closure System

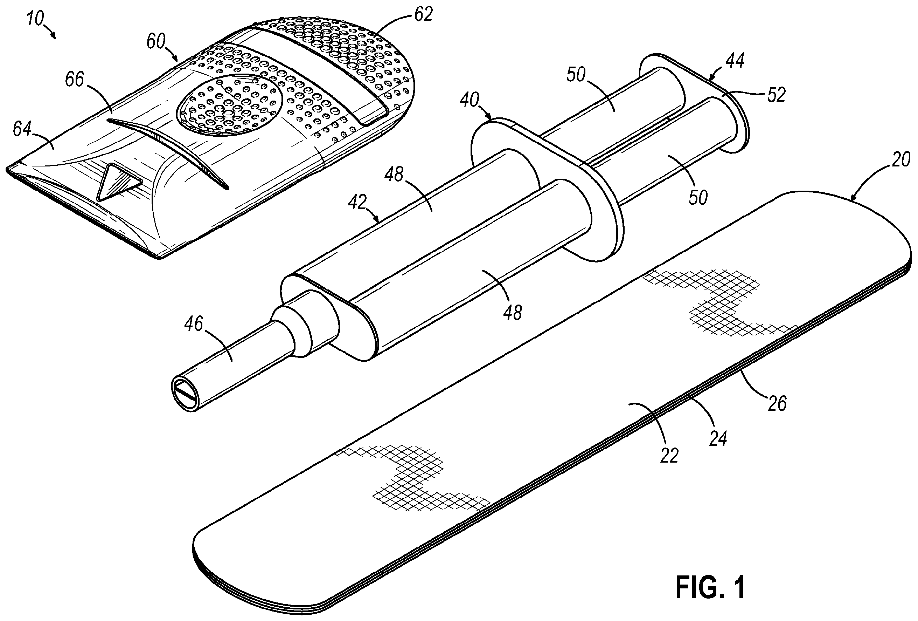

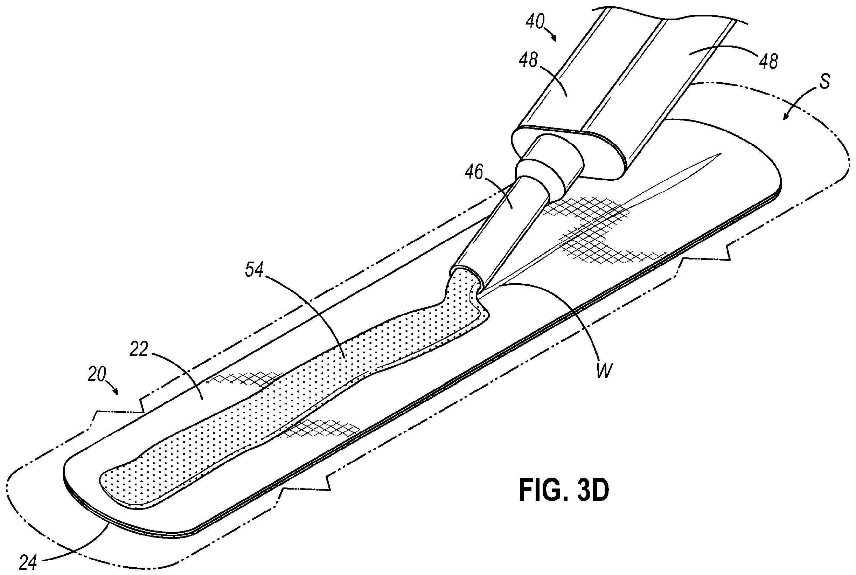

FIG. 1 shows an example of a wound closure system (10) that includes a wound closure device (20) (also referred to as a patch) configured to be applied to a patient's wound (W), an adhesive applicator (40) configured to apply a topical skin adhesive (54) (see FIG. 3D) to the applied wound closure device (20), and an adhesive spreader (60) configured to spread the applied topical skin adhesive (54) onto and through wound closure device (20). Each of these components is described in greater detail below.

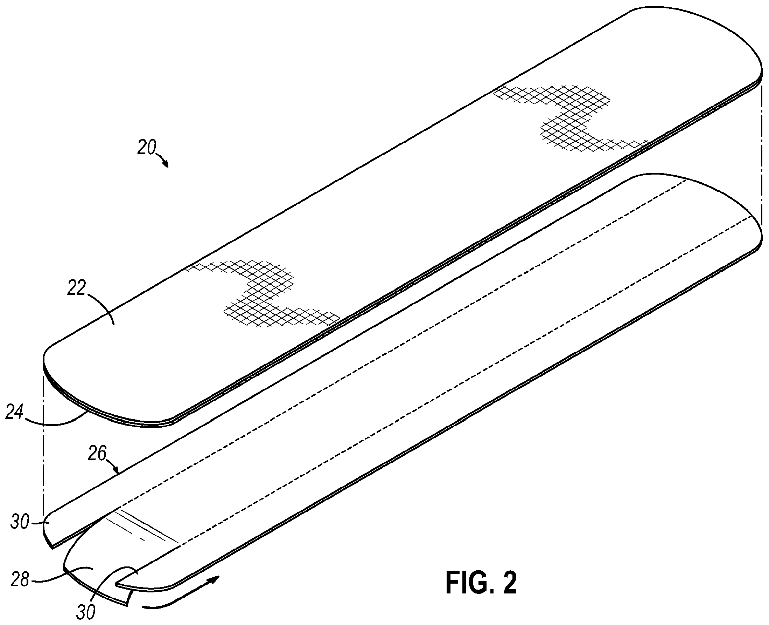

As shown best in FIG. 2, wound closure device (20) of the present version has an elongate, generally rectangular shape and a triple layer construction. More specifically, wound closure device (20) includes a layer of substrate in the form of a textile mesh (22), a layer of pressure sensitive adhesive (24) formed as a continuous or non-continuous coating along the lower skin-facing side of mesh (22), and a layer of backing (26) removably applied to the lower side of pressure sensitive adhesive (24). It will be understood that the term "wound closure device" as used herein encompasses wound closure device (20) both with and without backing (26), which may be removed and discarded during application of wound closure device (20) to a patient, as described in greater detail below.

Mesh (22) is configured to retain a liquid topical skin adhesive and may be formed of polyethylene (PET) or any other suitable surgical textile material. Pressure sensitive adhesive (24) is configured to enable wound closure device (20) to self-adhere to a patient's skin in response to a pressure being applied to the upper side of mesh (22) during its application by a surgeon. Backing (26) serves to protect pressure sensitive adhesive (24) before application of wound closure device (20) to the patient. In the present version, backing (26) includes elongate arrays of perforations that extend longitudinally and define an elongate central backing section (28) and a pair of elongate side backing sections (30). Though each backing section (28, 30) is shown as generally rectangular in the present version, backing sections (28, 30) may be of various alternative shapes, sizes, and quantities in other versions.

As shown in FIG. 1, adhesive applicator (40) includes an applicator body (42), a plunger unit (44) slidably received within an open proximal end of applicator body (42), and a static mixer (46) secured to a distal end of applicator body (42). Applicator body (42) includes a pair of barrels (48) arranged side by side, where each barrel (48) houses a respective part of a two-part liquid topical skin adhesive. Plunger unit (44) includes a pair of plungers (50) that are arranged side by side and are interconnected at their proximal ends by a bridge (52). Each plunger (50) is actuatable distally through a respective barrel (48) of applicator body (42) to force the corresponding liquid adhesive part distally into static mixer (46). Static mixer (46) is configured to receive the first and second adhesive parts and direct them around and through a series of static baffles and passages (not shown) that mix the two adhesive parts together into a homogenous liquid adhesive (54) that is then dispensed through an open distal end of static mixer (46), as shown in FIG. 3D described below.

In the present version, liquid topical skin adhesive (54) is in the form of a silicone-based topical skin adhesive that is configured to cure on skin at body temperature in less than two minutes. Once cured, topical skin adhesive (54) remains elastomeric such that a given section of cured adhesive (54) is configured to stretch up to 160% of its cured length and then fully recover to the cured length. Accordingly, wound closure system (10) may be particularly effective for use on actuatable body parts of a patient such as a knee, wrist, elbow, or other joint, for example.

As also shown in FIG. 1, adhesive spreader (60) of the present version has a monolithic body that includes a proximal body portion (62) configured to be gripped by a user, a distal body portion (64) that terminates at a tip configured to spread applied topical skin adhesive (54), and an intermediate flexural body portion (66) that interconnects the proximal and distal body portions (62, 64). Flexural body portion (66) is configured to elastically deform so that distal body portion (64) angularly deflects relative to proximal body portion (62) to promote effective spreading of topical skin adhesive (54) along wound closure device (20) with a suitable normal force within a predetermined range.

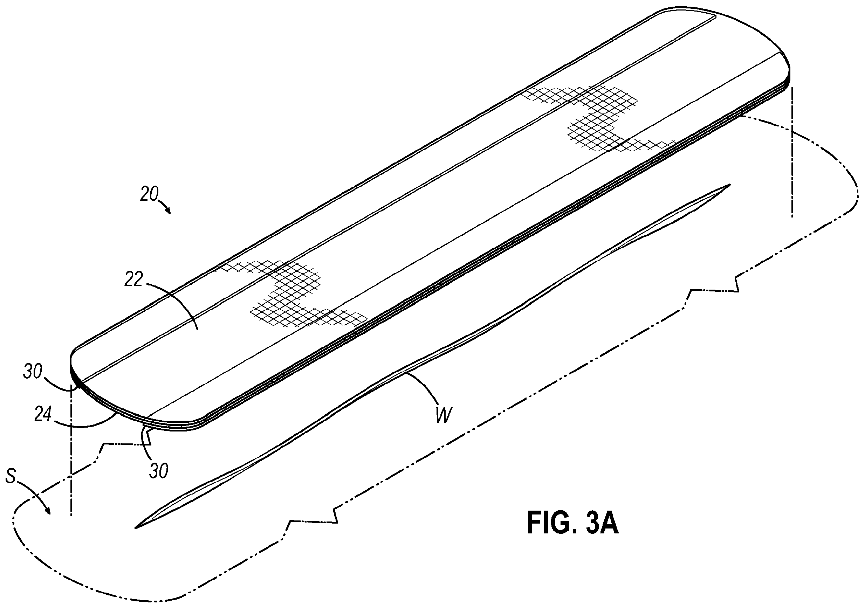

FIGS. 3A-3D show an example of wound closure system (10) being used to close a wound (W) formed in the skin (S) of a patient. In some procedures, wound (W) may be at least partially closed with one or more sutures, for example at deeper portions of wound (W), prior to use of wound closure system (10). Additionally, before wound closure device (20) is applied to the patient, it may be trimmed by a surgeon to any suitable shape as desired.

As shown in FIG. 3A, central backing section (28) is removed from wound closure device (20) to expose a central window of mesh (22) and pressure sensitive adhesive (24), and an imaginary centerline of wound closure device (20) is aligned longitudinally with the edges of wound (W). Wound closure device (20) is then applied to the patient skin (S) over wound (W) and the surgeon applies pressure to the central portion of mesh (22) to force pressure sensitive adhesive (24) to adhere to the skin (S), thus fixing the edges of wound (W) relative to one another. Before this step, the edges of wound (W) may be held in an approximated state by the surgeon. Alternatively, during this step the central portion of wound closure device (20) may be applied in a laterally alternating manner to approximate the edges of wound (W) during application. With either approach, wound closure device (20) is configured to hold the edges of wound (W) in an approximated state following this initial step of application. Once the surgeon is satisfied with the position of wound closure device (20) relative to wound (W), the surgeon may then remove the remaining two side backing sections (30) to adhere the remainder of wound closure device (20) to skin (S).

As shown in FIG. 3D, adhesive applicator (40) is then used to apply a pattern of topical skin adhesive (54) to the upper side of mesh (22) of the applied wound closure device (20). While adhesive applicator (40) is shown applying a linear bead of topical skin adhesive (54) in the present version, it will be appreciated that various other patterns of topical skin adhesive (54) may be applied in other versions, such as a T-shaped pattern or a wave-shaped pattern, as described in greater detail below.

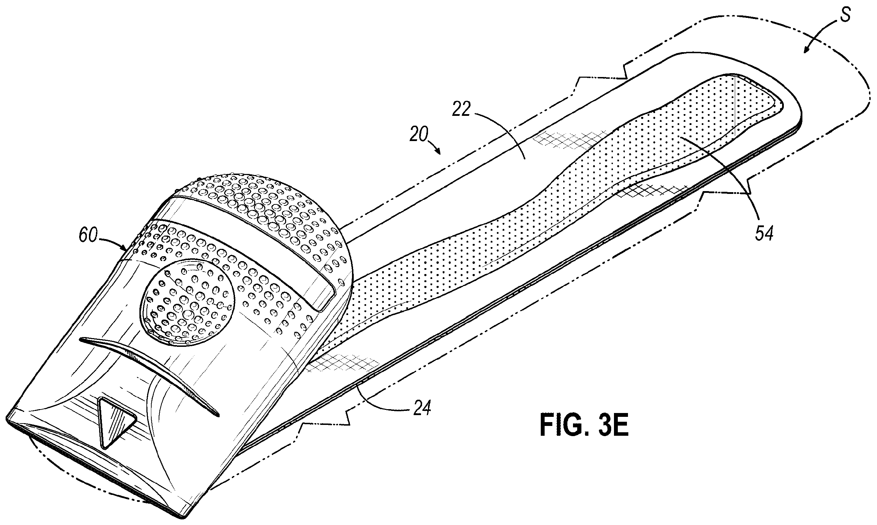

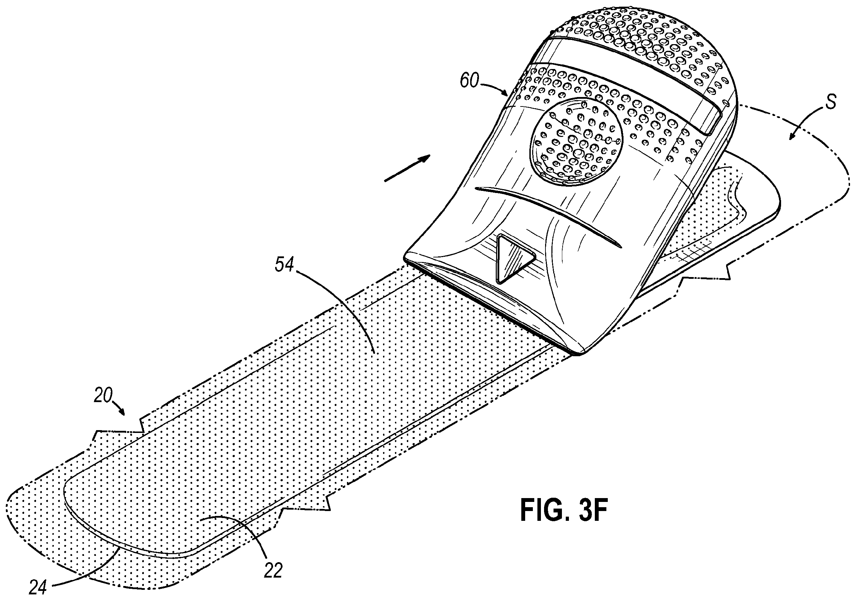

As shown in FIGS. 3E-3F, adhesive spreader (60) is then used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). In that regard, the layers of mesh (22) and pressure sensitive adhesive (24) may be at least partially permeable to permit forced passage of topical skin adhesive (54) therethrough. As shown in FIG. 3E, flexural body portion (66) of adhesive spreader (60) may be in a non-deformed state when adhesive spreader (60) is first positioned against wound closure device (20) at the beginning of an adhesive spreading stroke. As adhesive spreader (60) is dragged longitudinally along wound closure device (20), the input force exerted by the user may cause flexural body portion (66) to elastically deform such that distal body portion (64) angularly deflects relative to proximal body portion (62), as shown in FIG. 3F. The degree of deformation of flexural body portion (66) may be directly related to the viscosity of topical skin adhesive (54). In particular, a greater viscosity may require that the user exert a greater input force through proximal body portion (62) to effectively spread topical skin adhesive (54) over and through wound closure device (20), such that the flexural body portion (66) deforms a relatively greater amount. Conversely, a lesser viscosity may require a lesser input force such that the flexural body portion (66) deforms less or not at all.

Optionally, topical skin adhesive (54) may also be spread over adjacent portions of skin (S) not covered by wound closure device (20) to ensure that an entirety of mesh (22) is embedded with topical skin adhesive (54). For instance, and by way of example only, topical skin adhesive (54) may be spread onto at least 1 cm of skin (S) about the entire outer perimeter of the applied wound closure device (20). Once topical skin adhesive (54) has been fully spread over wound closure device (20), any topical skin adhesive (54) on skin (S) beyond the perimeter of device (20) may then be wiped away with sterile gauze, for example. Additionally, in some instances, a quantity of topical skin adhesive (54) may be applied between the edges of wound (W) before wound closure device (20) is applied to the skin (S). The applied topical skin adhesive (54) is then cured within and over wound closure device (20) to form a composite microbial barrier over wound (W) that maintains a protective environment that promotes efficient healing. Following healing of wound (W), wound closure device (20) may be removed from the skin (S) manually (e.g., by a surgeon) or it may automatically separate from the skin (S) such that it may be discarded by the patient.

Wound closure system (10) may be further configured and operable in accordance with at least some of the teachings of U.S. Pat. Pub. No. 2021/0369258, entitled "Systems, Devices and Methods for Dispensing and Curing Silicone Based Topical Skin Adhesives," published December 2, 2021; U.S. Pat. Pub. No. 2021/0371190, entitled "Systems, Methods and Devices for Aerosol Spraying of Silicone Based Topical Skin Adhesives for Sealing Wounds," published December 2, 2021; U.S. Pat. Pub. No. 2021/0371658, entitled "Novel Topical Skin Closure Compositions and Systems," published December 2, 2021; U.S. Pat. Pub. No. 20210369639, entitled "Novel Antimicrobial Topical Skin Closure Compositions and Systems," published December 2, 2021; U.S. Pat. Pub. No. 2021/0369276, entitled "Anisotropic Wound Closure Systems," published December 2, 2021; U.S. Pat. App. No. 17/667,950, entitled "Gas Sterilizable Syringes Having Apertures Covered by Gas Permeable Barriers for Enabling Ingress and Egress of Sterilization Gases While Preventing Leakage of Flowable Materials," filed on February 9, 2022; U.S. 17/991,992, entitled "Application of Topical Skin Adhesive to Surgical Mesh," filed on November 22, 2022; U.S. 17,991,945, entitled "Surgical Mesh Securing Device For Wound Closure System," filed on November 22, 2022; and/or U.S. 17/991,950, entitled "Wound Closure System Having Microcannulaic Pathways," filed on November 22, 2022.

### II. Illustrative Wound Closure Systems with Sensate

As mentioned above, wound closure device (20) is configured to be applied to a patient's wound (W) while topical skin adhesive (54) is configured to cure within and over wound closure device (20). Together, wound closure device (20) and cured adhesive (54) form a composite microbial barrier over wound (W); while cured adhesive (54) is suitably elastomeric such that a given section of cured adhesive (54) may stretch and fully recover to its cured length.

In some instances, it may be desirable to utilize a therapeutic agent that is suitable to interact with intact skin (S) (e.g., skin that is not damaged or sufficiently adjacent to an open sore/wound) of a patient, but not necessarily suitable to directly interact with injured skin (S) or wounds (W) of a patient; as such interaction may cause excessive pain and irritation of the wounded tissue. For example, various therapeutic agents that are not suitable for injured skin (S) or wounds (W) may be applied to intact skin (S) to provide various therapeutic effects that may improve the patient experience, such as alleviating pain, promoting accelerated healing, acting as a counterirritant, etc. In some instances, therapeutic agents may be applied to the skin (S) to evoke a specific desirable sensation by the patient (e.g., numbing, cooling, etc.). Unless stated otherwise, as used herein, a "sensate" is a therapeutic agent that is suitable for intact skin (S), but not suitable for injured skin (S) or wounds (W). For example, a sensate may be a therapeutic agent that is not suitable for injured skin (S) or wounds (W) but may be applied to intact skin (S) to provide various therapeutic effects that may improve the patient experience, such as alleviating pain, promoting accelerated healing, acting as a counterirritant, etc. In another example, a sensate may be a therapeutic agent that is suitable for intact skin (S), but not suitable for injured skin (S) or wounds (W), and evokes a specific desirable sensation by the patient (e.g., numbing, cooling, etc.). In the current application, some nonlimiting examples of sensate include the following: counterirritants, rubefacients that increase blood flow, vasodilators, cooling agents, irritants, local anesthetics, etc. Other sensate may include capsaicin, capsicum, lidocaine, menthol, methyl salicylate, other salicylates (e.g., glycol salicylate), topical forms of nonsteroidal anti-inflammatory drugs (NSAIDs), camphor, etc. While the term sensate is used in the examples described herein, such a term should not be limited to therapeutic agents that evoke a specific desirable sensation by a patient; but may also encompass therapeutic agents that may not be suitable for direct contact with injured skin (S) or wounds (W).

Therefore, it may be desirable to incorporate sensate into wound closure device (20), as such incorporation allows that sensate to interact with intact skin (S) within the composite microbial barrier to provide benefits to as would be apparent to one skilled in the art in view of the teachings herein (e.g., promoting blood flow to the general vicinity of wound (W), which may in turn promote efficient healing). However, in order to avoid undesirable consequences of sensate interacting directly with wound (or injured skin (S) adjacent to wound (W)), it may also be desirable to also limit, inhibit, and/or prevent the migration of such sensate from interacting with wound (W) (or injured skin (S) adjacent to wound (W)). In other words, allowing sensate to interact with intact skin (S); while limiting its ability to interact with wound (W) and/or injured skin (S) may enhance the patient experience and/or healing.

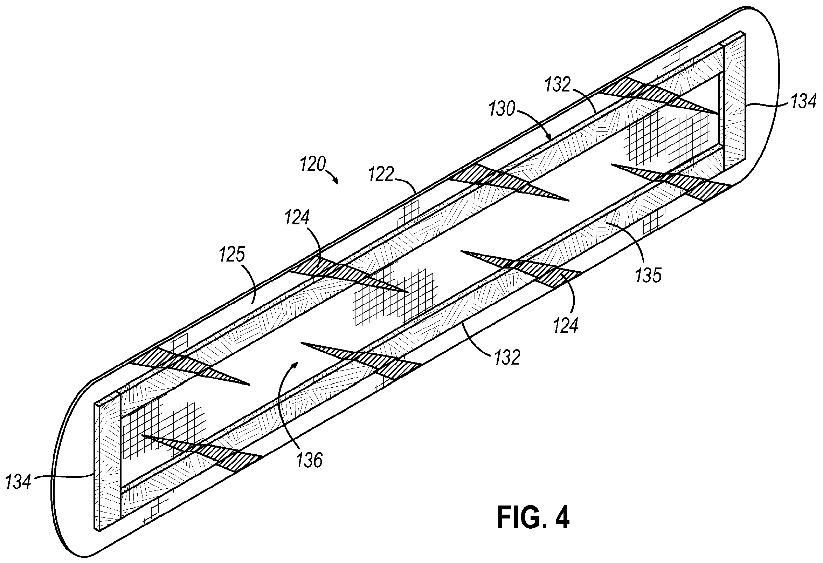

FIG. 4 depicts an illustrative wound closure device (120) that may be readily incorporated into wound closure system (10) in replacement of wound closure device (20), described above. Wound closure device (120) is substantially similar to wound closure device (20) described above, with differences elaborated herein. In particular, wound closure device (120) includes a sensate depot (130). As will be described in greater detail below, sensate depot (130) is incorporated with a suitable sensate (135) (or combination of multiple sensate (135)) that is configured to migrate and/or diffuse off depot (130) onto intact skin (S) within a composite microbial barrier formed by wound closure device (120) and topical skin adhesive (54); thereby providing therapeutic benefits within the microbial barrier. As will also be described in greater detail below, sensate depot (130) is sufficiently spaced away from the portion of wound closure device (120) intended to directly interact with wound (W) such that sensate (135) within the microbial barrier is inhibited from reaching and/or contacting wound (W) within the microbial barrier.

Wound closure device (120) includes a mesh (122) and a pressure sensitive adhesive (124), which may be substantially similar to mesh (22) and pressure sensitive adhesive (24) described above, with differences elaborated below. Therefore, pressure sensitive adhesive (124) is configured to allow wound closure device (120) to self-adhere to a patient skin in response to pressure being applied to an underside of wound closure device (120) during application by a surgeon. In the current example, pressure sensitive adhesive (124) is disposed on underside (125) of mesh (122) and select portions of sensate depot (130) in a "tiger stripe" pattern. Such a tiger stripe pattern may allow wound closure device (120) to self-adhere to a patient skin, while also exposing portions of underside (125) and sensate depot (130) directly to adjacent anatomy after wound closure device (120) is suitably applied on top of wound (W). Pressure sensitive adhesive (124) may be disposed on underside (125) of mesh (122) and sensate depot (130) in any suitable geometry as would be apparent to one skilled in the art in view of the teachings herein.

As will be described in greater detail below, in some instances, pressure sensitive adhesive (124) may be configured to dissolve after wound closure device (120) is suitably applied to wound (W) and a microbial barrier is formed, thereby exposing portions of sensate depot (130) to intact skin (S) for migration/disbursement of sensate onto skin (S). As will also be described in greater detail below, in some instances, pressure sensitive adhesive (124) may be configured to allow sensate (135) to diffuse through pressure sensitive adhesive (124) after wound closure device (120) is suitably applied to wound (W) and a microbial barrier is formed, thereby allowing sensate (135) to interact with intact skin (S) even if pressure sensitive adhesive (124) is directly interposed between sensate depot (130) and intact skin (S).

While not shown, it should be understood that wound closure device (120) may also include a removable backing section, which may temporarily cover selected surfaces (including sensate depot (130) described below) in substantially similar manner to backing (26) described above. Therefore, such a backing section may serve to protect pressure sensitive adhesive (124) and sensate depot (130) before application of wound closure device (120) to the patient.

While wound closure device (120) in the current example includes a body in the form of textile mesh (122) in the current example, it should be understood that such a body of wound closure device (120) may be formed with any other suitable material as would be apparent to one skilled in the art in view of the teaching herein. For example, instead of mesh (122), such a body of wound closure device (120) may be formed of a perforated fabric, a woven fabric, a non-woven fabric, a film material, etc.

Wound closure device (120) also includes sensate depot (130) attached to textile mesh (122). As mentioned above, sensate depot (130) has diffusible/migratable sensate (135) incorporated therein. Sensate (135) associates with depot (130) such that after wound closure device (120) is adhered to a patient in accordance with the description herein, sensate (135) migrates and/or diffuses off depot (130) and toward intact skin (S). Therefore, sensate depot (130) allows wound closure device (120) to apply sensate (135) onto intact skin (S) within the microbial barrier in order to provide therapeutic benefits. In some instances, direct contact between sensate depot (130) and skin (S) causes sensate to diffuse/migrate off depot (130) and onto skin (S). In some instances, exposure to moisture, such as bodily fluids, may encourage sensate (135) to migrate and/or diffuse off depot (130). In some instances, exposure to a temperature above or below a threshold temperature may encourage sensate (135) to migrate and/or diffuse off depot (130).

Sensate depot (130), in the current example, includes a pair of elongated longitudinal bodies (132) located on opposing lateral sides of underside (125) of mesh (122); and a pair of terminating elongated lateral bodies (134) connecting longitudinal ends of respective elongated longitudinal bodies (132). In the current example, bodies (132, 134) extend downwardly from underside (125) of mesh (122) and together form a generally rectangular shape/perimeter. However, bodies (132, 134) of sensate depot (130) may be located at any other suitable location relative to mesh (122) as would be apparent to one skilled in the art in view of the teachings herein. While elongated bodies (132, 134) are used to form a generally rectangular sensate depot (130) in the current example, sensate depot (130) may include any suitably geometry as would be apparent to one skilled in the art in view of the teachings herein. Bodies (132, 134) may be formed of a suitable material capable of housing sensate (135), and also allowing sensate (135) to diffuse/migrate in accordance with the description herein.

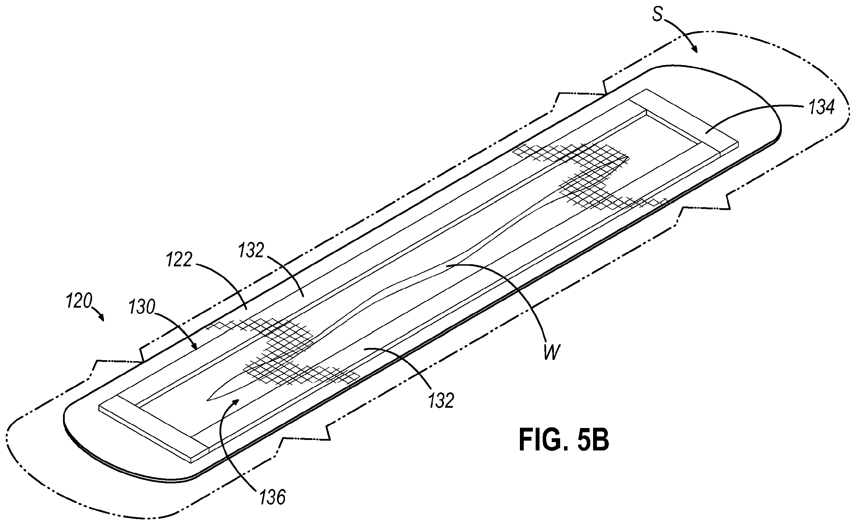

Together, elongated longitudinal bodies (132) and elongated lateral bodies (134) define an isolated wound area (136). Isolated wound area (136) is suitably large enough to contain wound (W) within its perimeter *(see* FIG.5B) such that wound (W) is sufficiently spaced away from sensate depot (130) when wound closure device (120) is adhered to skin (S) in accordance with the teachings herein. Therefore, when sensate (135) migrates/diffuses off depot (130) and onto skin (S) in accordance with the description herein, isolated wound area (136) may help inhibit sensate (135) from directly interacting with wound (W).

As will be described in greater detail below, after topical skin adhesive (54) is applied onto/into wound closure device (120) in accordance with the description herein, a portion of topical skin adhesive (54) may cure between sensate depot (130) and wound (W). Therefore, cured topical skin adhesive (54), as well as the size of isolated wound area (136), may inhibit sensate from migrating off of depot (130) into wound (W) after the composite microbial barrier is formed in accordance with teachings herein.

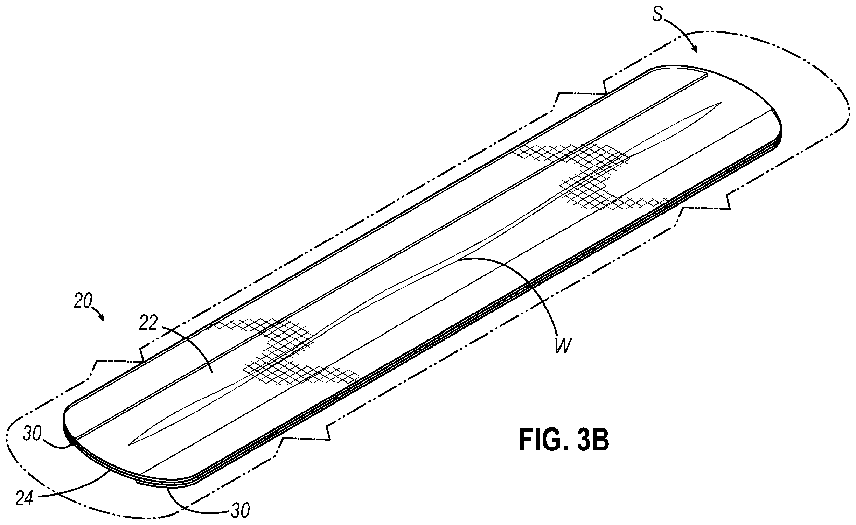

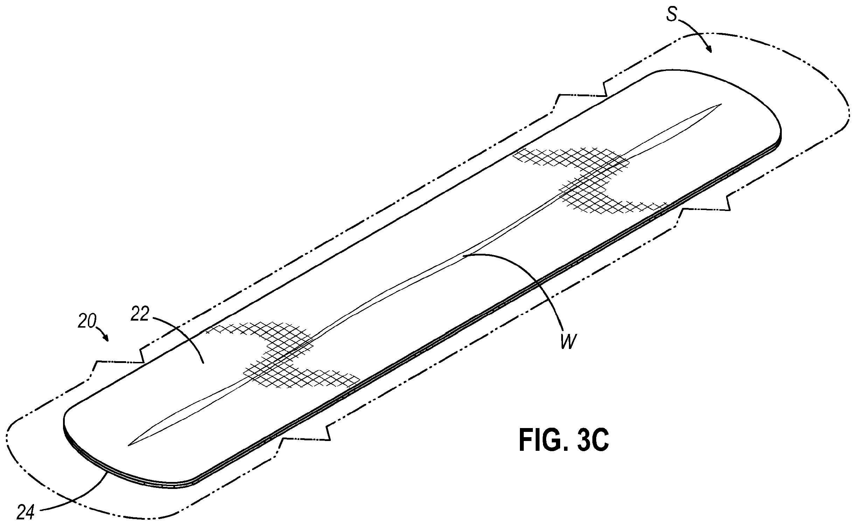

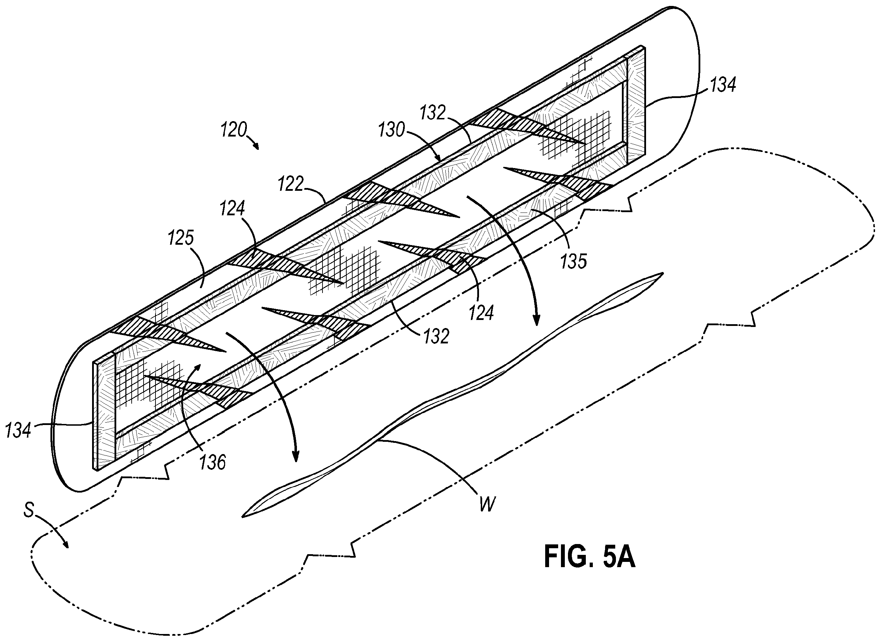

FIGS. 5A-5E show an example use of wound closure device (120) with suitable components of wound closure system (10) in order to close a wound (W) formed in the skin (S) of patient. In particular, wound closure device (120) is used in replacement of wound closure device (20) described above. As shown in FIG. 5A, wound closure device (120) may be positioned over skin (S) and wound (W) of patent. It should be understood that while FIG. 5A shows wound closure device (120) rotated such that underside (125) is not facing directly toward skin (S), wound closure device (120) may be positioned above wound (W) and skin (S) such that underside (125) faces wound (W) (similar to the position shown in FIG. 3), and isolated wound area (136) is directly adjacent to wound (W).

Next, as shown in FIG. 5B, wound closure device (120) is then applied to the patient skin (S) over wound (W) and the surgeon applies pressure to mesh (122) to force pressure sensitive adhesive (124) to adhere to the skin (S), thus fixing the edges of wound (W) relative to one another. As also shown in FIG. 5B, sensate depot (130) is positioned such that wound (W) is housed within isolated won area (136). Therefore, sensate depot (130) surrounds wound (W) with a suitable amount of distance between bodies (132, 134) and wound (W). As mentioned above, isolated wound area (136) may help inhibit sensate (135) **migrating/diffusing** off depot (130) and toward skin (S) from directly interacting with wound (W).

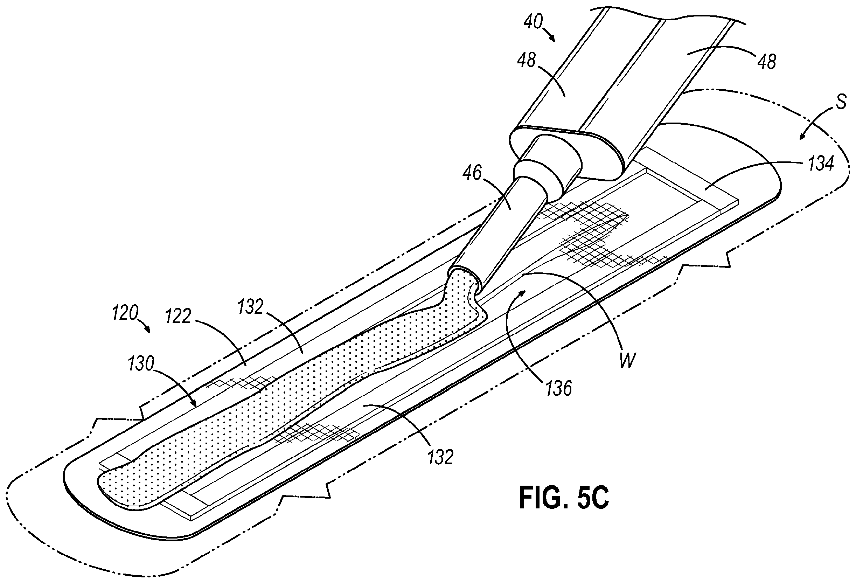

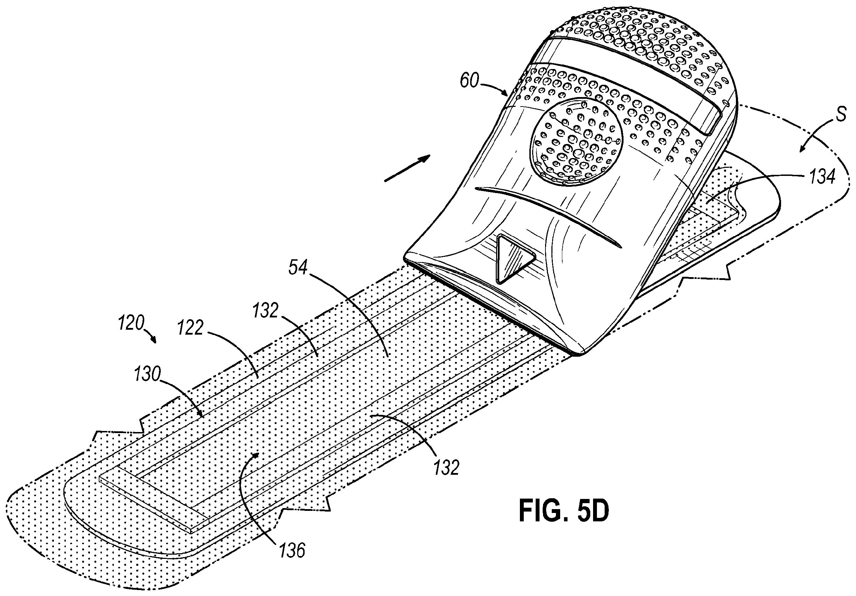

As shown in FIG. 5C, adhesive applicator (40) is then used to apply a pattern of topical skin adhesive (54) to the upper side of mesh (122) of the applied wound closure device (120). As shown in FIGS. 5D, adhesive spreader (60) is then used to spread the applied topical skin adhesive (54) uniformly over wound closure device (120) to force the topical skin adhesive (54) through the layers of mesh (122) and pressure sensitive adhesive (124), within isolated wound area (136), and directly against wound (W) and the surrounding skin (S). After a suitable amount of time (e.g., two minutes), topical skin adhesive (54) may cure, thereby forming of composite microbial barrier over wound (W). Additionally, cured topical sensitive adhesive (54) within isolated wound area (136) may act as an additional barrier that inhibits sensate (135) from diffusing off depot (130) and toward wound (W).

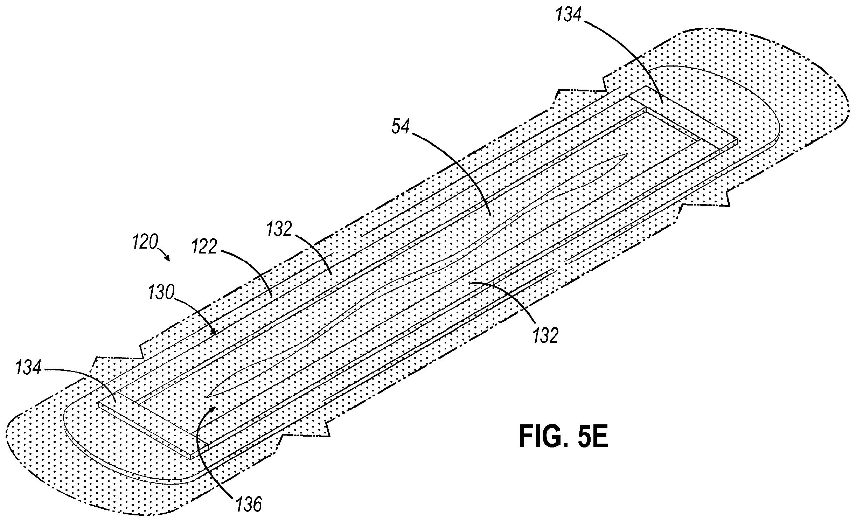

With wound closure device (120) and topical skin adhesive (54) forming the composite microbial barrier shown in FIG. 5E, sensate (135) may diffuse off depot (130) and onto intact skin (S), thereby providing various therapeutic advantages as would be apparent to one skilled in the art in view of the teachings herein. Further, the distance between depot (130) and wound (W), and/or the presence of cured topical skin adhesive (58) within isolated wound area (136) inhibits sensate (135) from undesirably interacting with wound (W).

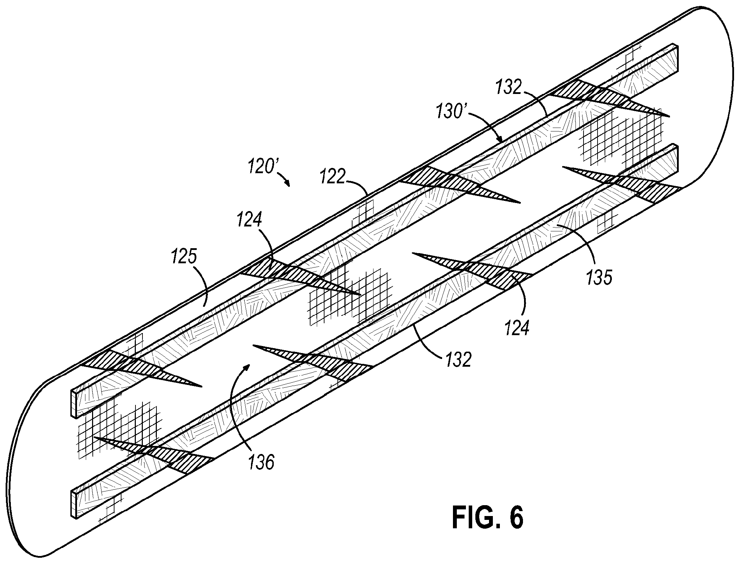

As mentioned above, sensate depot (130) may have any suitable geometry and be located at any suitable location relative to mesh (122) as would be apparent to one skilled in the art in view of the teachings herein. FIGS. 6-11 show various alternative examples of wound closure devices (120', 140, 160, 180, 200, 220) with respective sensate depots (130', 150, 170, 190, 210, 230).

FIG. 6 shows wound closure device (120') that is substantially similar to wound closure device (120) described above, with differences elaborated herein. Therefore, wound closure device (120') may be readily incorporated into wound closure system (10) in replacement of wound closure device (20 ,120) described above. Sensate depot (130') of wound closure device (120') has a pair of elongated longitudinal bodies (132) located on opposing lateral sides of underside (125) of mesh (122); but does not include any type of elongated lateral bodies connected terminating ends of longitudinal bodies (132). Therefore, in the present example, longitudinal bodies (132) define isolated wound area (136) but are not connected to each other to form a continuous perimeter.

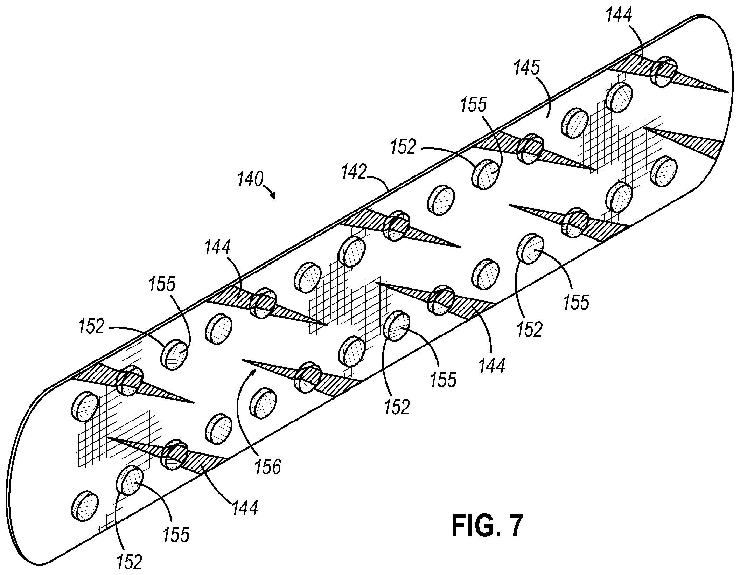

FIG. 7 shows wound closure device (140) that is substantially similar to wound closure device (120, 120') described above, with differences elaborated herein. Therefore, wound closure device (140) may be readily incorporated into wound closure system (10) in replacement of wound closure device (20, 120, 120') described above. Wound closure device (140) includes a textile mesh (142), pressure sensitive adhesive (144), and an underside (145); which may be substantially similar to textile mesh (122), pressure sensitive adhesive (124), and underside (125) described above, with differences elaborated herein.

Wound closure device (140) includes sensate depots (150) in the form of discrete bodies (152) arranged in two respective linear arrays, each linear array located on opposing lateral sides of underside (145). Each body (152) is incorporated with sensate (155) in substantially similar fashion as bodies (132, 134) and sensate (135) described above. Therefore, sensate (155) diffuses/migrates away from its respective body (152) onto intact skin (S) after the formation of a composite microbial barrier in order to provide therapeutic effects. The opposing linear arrays of sensate depots (150) defines an isolated wound area (156); which may function in substantially similar manner as isolated wound areas (136) described above. Therefore, sensate depots (150) may be formed by a plurality of bodies (152) located on eh same lateral side of mesh (142).

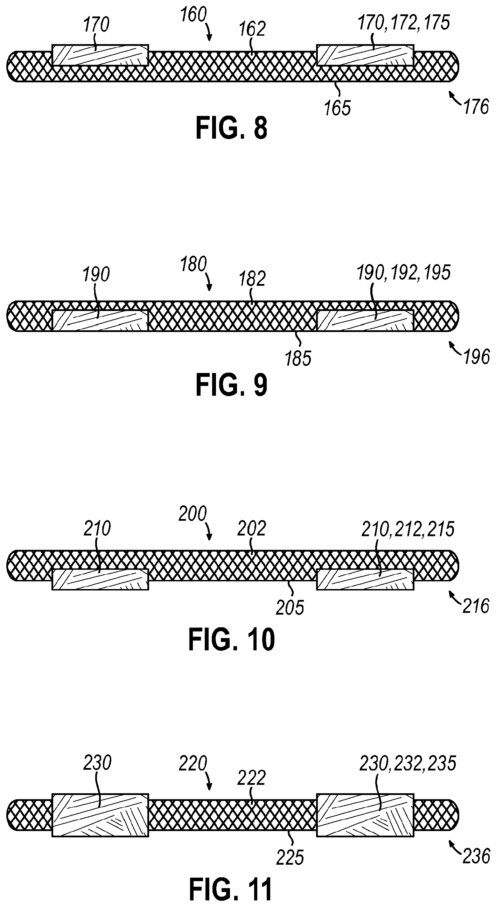

FIG. 8 shows wound closure device (160) that is substantially similar to wound closure device (120, 120', 140) described above, with differences elaborated herein. Therefore, wound closure device (160) may be readily incorporated into wound closure system (10) in replacement of wound closure device (20, 120, 120', 140) described above. Wound closure device (160) includes a textile mesh (162), pressure sensitive adhesive (not shown), and an underside (165); which may be substantially similar to textile mesh (122, 142), pressure sensitive adhesive (124, 144), and underside (125, 145) described above, with differences elaborated herein.

Wound closure device (160) includes sensate depots (170) in the form of bodies (172) located on opposing lateral sides of mesh (162). Each body (172) is incorporated with sensate (175) in substantially similar fashion as bodies (132, 134, 152) and sensate (135, 155) described above. Therefore, sensate (175) diffuses/migrates away from body (172) onto intact skin (S) after the formation of a composite microbial barrier in order to provide therapeutic effects. In the current example, each body (172) is located above the underside (165) of mesh (162). Therefore, sensate (175) may be configured to diffuse through portions of mesh (162) located between body (172) and intact skin (S) in order to suitable reach intact skin (S). Sensate (175) may reach intact skin (S) using any suitable technique as would be apparent to one skilled in the art in view of the teachings herein. The laterally opposing sensate depots (170) define an isolated wound area (176); which may function in substantially similar manner as isolated wound areas (136, 156) described above.

FIG. 9 shows wound closure device (180) that is substantially similar to wound closure device (120, 120', 140, 160) described above, with differences elaborated herein. Therefore, wound closure device (180) may be readily incorporated into wound closure system (10) in replacement of wound closure device (20, 120, 120', 140, 160) described above. Wound closure device (180) includes a textile mesh (182), pressure sensitive adhesive (not shown), and an underside (185); which may be substantially similar to textile mesh (122, 142, 162), pressure sensitive adhesive (124, 144), and underside (125, 145, 165) described above, with differences elaborated herein.

Wound closure device (180) includes sensate depots (190) in the form of bodies (192) located on opposing lateral sides of mesh (182). Each body (192) is incorporated with sensate (195) in substantially similar fashion as bodies (132, 134, 152, 172) and sensate (135, 155, 175) described above. Therefore, sensate (195) diffuses/migrates away from body (192) onto intact skin (S) after the formation of a composite microbial barrier in order to provide therapeutic effects. In the current example, each body (192) is located within mesh (182) and flush with underside (185). Therefore, sensate (195) may be configured to migrate onto suitable portions of intact skin (S) while also allowing portions of underside (185) directly adjacent to body (192) to engage skin (S). The laterally opposing sensate depots (190) define an isolated wound area (196); which may function in substantially similar manner as isolated wound areas (136, 156, 176) described above.

FIG. 10 shows wound closure device (200) that is substantially similar to wound closure device (120, 120', 140, 160, 180) described above, with differences elaborated herein. Therefore, wound closure device (200) may be readily incorporated into wound closure system (10) in replacement of wound closure device (20, 120, 120', 140, 160, 180) described above. Wound closure device (200) includes a textile mesh (202), pressure sensitive adhesive (not shown), and an underside (205); which may be substantially similar to textile mesh (122, 142, 162, 182), pressure sensitive adhesive (124, 144), and underside (125, 145, 165, 185) described above, with differences elaborated herein.

Wound closure device (200) includes sensate depots (210) in the form of bodies (212) located on opposing lateral sides of mesh (202). Each body (212) is incorporated with sensate (215) in substantially similar fashion as bodies (132, 134, 152, 172, 192) and sensate (135, 155, 175, 195) described above. Therefore, sensate (215) diffuses/migrates away from body (212) onto intact skin (S) after the formation of a composite microbial barrier in order to provide therapeutic effects. In the current example, each body (212) is located within mesh (202) and partially extending downwardly from underside (205). Therefore, sensate (215) may be configured to migrate onto suitable portions of intact skin (S). The laterally opposing sensate depots (210) define an isolated wound area (216); which may function in substantially similar manner as isolated wound areas (136, 156, 176, 196) described above.

FIG. 11 shows wound closure device (220) that is substantially similar to wound closure device (120, 120', 140, 160, 180, 200) described above, with differences elaborated herein. Therefore, wound closure device (220) may be readily incorporated into wound closure system (10) in replacement of wound closure device (20, 120, 120', 140, 160, 180, 200) described above. Wound closure device (220) includes a textile mesh (222), pressure sensitive adhesive (not shown), and an underside (225); which may be substantially similar to textile mesh (122, 142, 162, 182, 202), pressure sensitive adhesive (124, 144), and underside (125, 145, 165, 185, 205) described above, with differences elaborated herein.

Wound closure device (220) includes sensate depots (230) in the form of bodies (232) located on opposing lateral sides of mesh (222). Each body (232) is incorporated with sensate (235) in substantially similar fashion as bodies (132, 134, 152, 172,192, 212) and sensate (135, 155, 175, 195, 215) described above. Therefore, sensate (235) diffuses/migrates away from body (232) onto intact skin (S) after the formation of a composite microbial barrier in order to provide therapeutic effects. In the current example, each body (232) is located within mesh (222) and partially extends both upwardly and downwardly from mesh (222). Therefore, sensate (235) may be configured to migrate onto suitable portions of intact skin (S). The laterally opposing sensate depots (230) define an isolated wound area (236); which may function in substantially similar manner as isolated wound areas (136, 156, 176, 196, 216) described above.

As mentioned above, and as shown in FIG. 5D, adhesive spreader (60) is used to spread the applied topical skin adhesive (54) uniformly over wound closure device (120) to force the topical skin adhesive (54) through the layers of mesh (122) and pressure sensitive adhesive (124) and directly against wound (W) and the surrounding skin (S). In some instances, while topical skin adhesive (54) is being forced through layer of mesh (122) and/or pressures sensitive adhesive (124), adhesive (54) may cure between depot (130) and intact skin (S) directly adjacent to depot (130). If adhesive (54) cures between sensate depot (130) and intact skin (S) directly adjacent thereto, sensate (135) may be undesirably inhibited from diffusing/migrating onto intact skin (S), which may limit the ability for sensate (135) to provide desirable therapeutic treatments to intact skin (S) after the formation of the composite microbial barrier. Therefore, it may be desirable to further inhibit topical skin adhesive (54) from curing between sensate depot (130) and intact skin (S) in order to promote suitable diffusion/migration of sensate (135) onto intact skin (S).

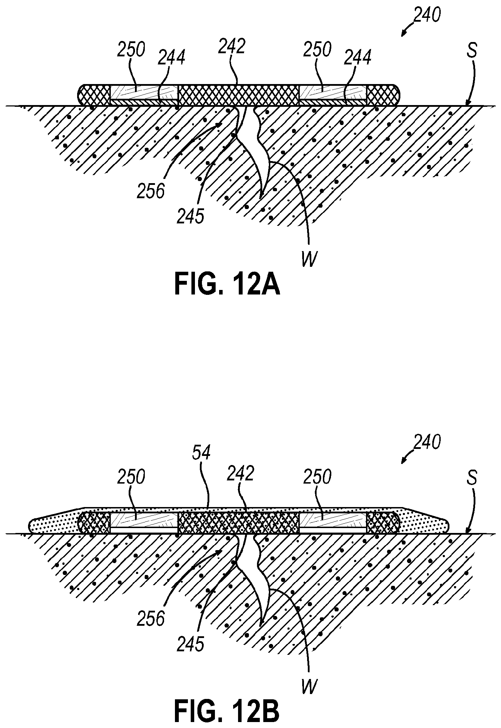

FIGS. 12A-12B show an alternative wound closure device (240) that is substantially similar to wound closure device (120, 120', 140, 160, 180, 200, 220) described above, with differences elaborated herein. Therefore, wound closure device (240) may be readily incorporated into wound closure system (10) in replacement of wound closure device (20, 120, 120', 140, 160, 180, 200, 220) described above. Wound closure device (240) is configured to inhibit topical skin adhesive (54) from curing between an underside (245) of mesh (242) and intact skin (S) in order to promote suitable diffusion/migration of sensate onto intact skin (S). Wound closure device (240) includes a textile mesh (242), pressure sensitive adhesive (244), an underside (245), and sensate depot (250); which may be substantially similar to textile mesh (122, 142, 162, 182, 202, 222), pressure sensitive adhesive (124, 144), underside (125, 145, 165, 185, 205, 225), and sensate depot (130, 150, 170, 190, 210, 230) described above, with differences elaborated herein.

As best shown in FIG. 12A, pressure sensitive adhesive (244) is disposed on the underside of sensate depot (250). Pressure sensitive adhesive (244) may extend coextensively with the underside of sensate depot (250) such that when wound closure device (240) in initially self-adhered onto skin (S) in accordance with the description herein, pressure sensitive adhesive (244) is interposed between the underside of sensate depot (250) and intact skin (S). In some instances, rather than extending coextensively, pressure sensitive adhesive (244) may extend along a substantial and/or suitable portion of the underside of sensate depot (250) in order to allow suitable commination between sensate depot (250) and intact skin (S) after the formation of the composite microbial barrier in accordance with the description herein. Pressure sensitive adhesive (244) allows a surgeon to self-adhere wound closure device (240) onto skin (S) of wound (W) in substantially similar fashion to pressure sensitive adhesive (24, 124, 144) described above.

In the current example, pressure sensitive adhesive (244) is soluble such that pressures sensitive adhesive is configured to dissolve in response to exposure to fluids. Therefore, after a suitable amount of time being exposed to skin (S) of patient, pressure sensitive adhesive (244) dissolves, leaving a communications pathway adjacent to sensate depot (250). However, prior to dissolving, pressure sensitive adhesive (244) acts as a protective barrier between sensate depot (250) and directly adjacent portions of intact skin (S), as shown in FIG. 12A.

During application of topical skin adhesive (54), such topical adhesive (54) penetrates mesh (242) in order to cure between underside (245) of mesh and intact skin (S) directly adjacent to corresponding portions of underside (245). However, pressure sensitive adhesive (244), acting as a barrier, prevents uncured topical skin adhesive (54) from reaching portions of intact skin (S) directly under sensate depot (250). Therefore, topical skin adhesive (54) is inhibited from curing onto intact skin (S) directly under sensate depots (250). Because pressure sensitive adhesive (244) is dissolvable; after the formation of the microbial barrier, as shown in FIG. 12B, pressure sensitive adhesive (244) dissolves in response to exposure to bodily fluids, thereby creating a fluid communication path between intact skin (S) and sensate depot (250).

Sensate depots (250) are also laterally spaced from the lateral center of mesh (242) directly adjacent to wound (W) such that wound closure device (240) defines an isolated wound area (256). Additionally, the portions of topical skin adhesive (54) that cure on the underside (245) of mesh (242) between wound (W) and sensate depot (250) create a sealed barrier inhibiting sensate from undesirably reaching wound (W). Therefore, soluble pressures sensitive adhesive (244) ensures sensate depot (250) may deliver sensate to suitable portions of intact skin (S), while cured topical skin adhesive (54) inhibits undesirable migration of sensate towards wound (W).

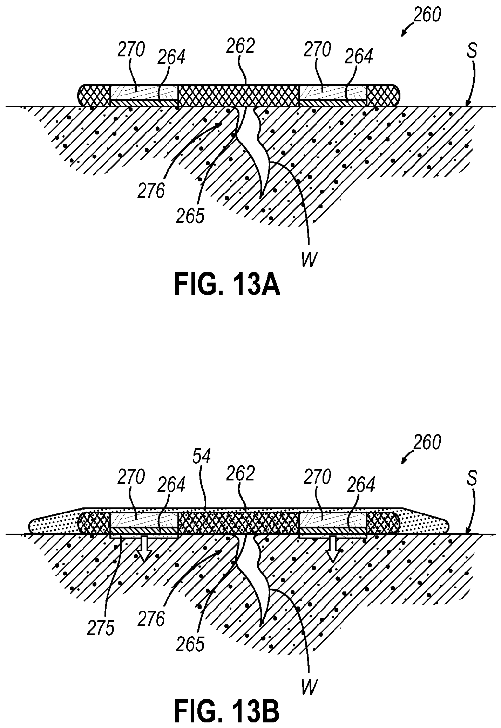

FIGS. 13A-13B show an alternative wound closure device (260) that is substantially similar to wound closure device (120, 120', 140, 160, 180, 200, 220, 240) described above, with differences elaborated herein. Therefore, wound closure device (260) may be readily incorporated into wound closure system (10) in replacement of wound closure device (20, 120, 120', 140, 160, 180, 200, 220, 240) described above. Wound closure device (260) is configured to inhibit topical skin adhesive (54) from curing between an underside (265) of mesh (262) and intact skin (S) in order to promote suitable diffusion/migration of sensate onto intact skin (S). Wound closure device (260) includes a textile mesh (262), pressure sensitive adhesive (264), an underside (265), and sensate depot (270); which may be substantially similar to textile mesh (122, 142, 162, 182, 202, 222, 242), pressure sensitive adhesive (124, 144, 244), underside (125, 145, 165, 185, 205, 225, 245), and sensate depot (130, 150, 170, 190, 210, 230, 250) described above, with differences elaborated herein.

As best shown in FIG. 13A, pressure sensitive adhesive (264) is disposed on the underside of sensate depot (270). Pressure sensitive adhesive (264) may extend coextensively with the underside of sensate depot (270) such that when wound closure device (260) in initially self-adhered onto skin (S) in accordance with the description herein, pressure sensitive adhesive (264) is interposed between the underside of sensate depot (270) and intact skin (S). In some instances, rather than extending coextensively, pressure sensitive adhesive (264) may extend along a substantial and/or suitable portion of the underside of sensate depot (270) in order to allow suitable commination between sensate depot (270) and intact skin (S) after the formation of the composite microbial barrier in accordance with the description herein. Pressure sensitive adhesive (264) allows a surgeon to self-adhere wound closure device (260) onto skin (S) of wound (W) in substantially similar fashion to pressure sensitive adhesive (24, 124, 144, 244) described above.

As mentioned above, pressure sensitive adhesive (264) is substantially similar to pressure sensitive adhesive (244) described above, with differences elaborated herein. In particular, rather than being soluble, pressure sensitive adhesives (264) is formed of a suitable material that allows sensate (275) to travel through pressure sensitive adhesive (264) (e.g., diffuse through, penetrate through, etc.,) from sensate depot (270) onto intact skin (S) directly under pressure sensitive adhesive (264).

Additionally, pressures sensitive adhesive (264) is configured to inhibit liquid topical skin adhesive (54) from penetrating into pressures sensitive adhesive (264). Therefore, as topical adhesive (54) is applied, pressure sensitive adhesive (264) acts as a protective barrier between sensate depot (270) and directly adjacent portions of intact skin (S), thereby inhibiting topical adhesive (54) from reaching a position directly interposed between pressures sensitive adhesive (264) and skin (S) in direct contact with pressure sensitive adhesives (264).

During application of topical skin adhesive (54), such topical adhesive (54) penetrates mesh (262) in order to cure between underside (265) of mesh and intact skin (S) directly adjacent to corresponding portions of underside (265). However, pressure sensitive adhesive (264), acting as a barrier, prevents uncured topical skin adhesive (54) from reaching portions of intact skin (S) directly under sensate depot (270). Therefore, topical skin adhesive (54) is inhibited from curing onto intact skin (S) directly under sensate depots (270). Because pressure sensitive adhesive (264) allows sensate (275) to travel through pressures sensitive adhesive (264); after the formation of the microbial barrier, as shown in FIG. 12B, pressure sensitive adhesive (264) accommodates a fluid communication path between intact skin (S) and sensate depot (270).

Sensate depots (270) are also laterally spaced from the lateral center of mesh (262) directly adjacent to wound (W) such that wound closure device (260) defines an isolated wound area (276). Additionally, the portions of topical skin adhesive (54) that cure on the underside (265) of mesh (262) between wound (W) and sensate depot (270) create a sealed barrier inhibiting sensate (275) from undesirably reaching wound (W). Therefore, pressure sensitive adhesive (264) ensures sensate depot (270) may deliver sensate to suitable portions of intact skin (S), while cured topical skin adhesive (54) inhibits undesirable migration of sensate towards wound (W).

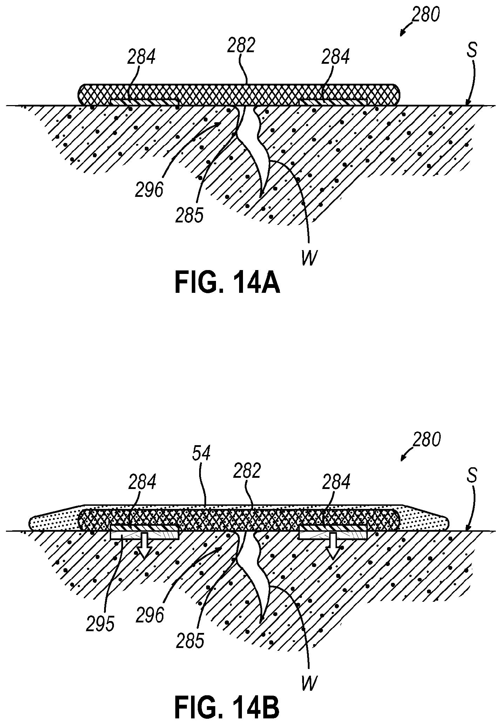

FIGS. 14A-14B show an alternative wound closure device (280) that is substantially similar to wound closure device (120, 120', 140, 160, 180, 200, 220, 240, 260) described above, with differences elaborated herein. Therefore, wound closure device (280) may be readily incorporated into wound closure system (10) in replacement of wound closure device (20, 120, 120', 140, 160, 180, 200, 220, 240, 260) described above. Wound closure device (280) is configured to inhibit topical skin adhesive (54) from curing between an underside (285) of mesh (282) and intact skin (S) in order to promote suitable diffusion/migration of sensate (295) onto intact skin (S). Wound closure device (280) includes a textile mesh (282), pressure sensitive adhesive (284), and an underside (285); which may be substantially similar to textile mesh (122, 142, 162, 182, 202, 222, 242, 262), pressure sensitive adhesive (124, 144, 244, 264), and underside (125, 145, 165, 185, 205, 225, 245, 265), described above, with differences elaborated herein.

In the current example, pressure sensitive adhesive (284) serves as a means for self-adhering wound closure device (280) to skin (S). Additionally, pressure sensitive adhesive (284) also functions as a sensate depot, suitably housing sensate (295) in substantially similar manner as sensate depot sensate depot (130, 150, 170, 190, 210, 230, 250) described above. Therefore, pressure sensitive adhesive (284) is incorporated with a suitable sensate (295) (or combination of multiple sensate (295)) that is configured to migrate and/or diffuse off pressure sensitive adhesive (284) onto intact skin (S) within a composite microbial barrier formed by wound closure device (280) and topical skin adhesive (54); thereby providing therapeutic benefits within the microbial barrier. As such, pressure sensitive adhesive (284) is laterally spaced from the lateral center of mesh (282) directly adjacent to wound (W) such that wound closure device (280) defines an isolated wound area (296). Pressure sensitive adhesives (284) may be applied to underside (285) with any suitable pattern/geometry as would be apparent to one skilled in the art in view of the teachings herein.

Additionally, pressures sensitive adhesive (284) is configured to inhibit liquid topical skin adhesive (54) from penetrating into pressure sensitive adhesive (284). Therefore, as topical adhesive (54) is applied, pressure sensitive adhesive (284) acts as a protective barrier between pressure sensitive adhesive (284) and directly adjacent portions of intact skin (S), thereby inhibiting topical adhesive (54) from reaching a position directly interposed between pressures sensitive adhesive (284) and skin (S) in direct contact with pressure sensitive adhesives (284).

During application of topical skin adhesive (54), such topical adhesive (54) penetrates mesh (282) in order to cure between underside (285) of mesh and intact skin (S) directly adjacent to corresponding portions of underside (285). However, pressure sensitive adhesive (284), acting as a barrier, prevents uncured topical skin adhesive (54) from reaching portions of intact skin (S) directly under pressure sensitive adhesive (284). Additionally, the portions of topical skin adhesive (54) that cure on the underside (285) of mesh (282) between wound (W) and pressure sensitive adhesive (284) create a sealed barrier inhibiting sensate (295) from undesirably reaching wound (W). Therefore, pressure sensitive adhesive (284) ensures sensate (295) may be delivered suitable portions of intact skin (S), while cured topical skin adhesive (54) inhibits undesirable migration of sensate towards wound (W).

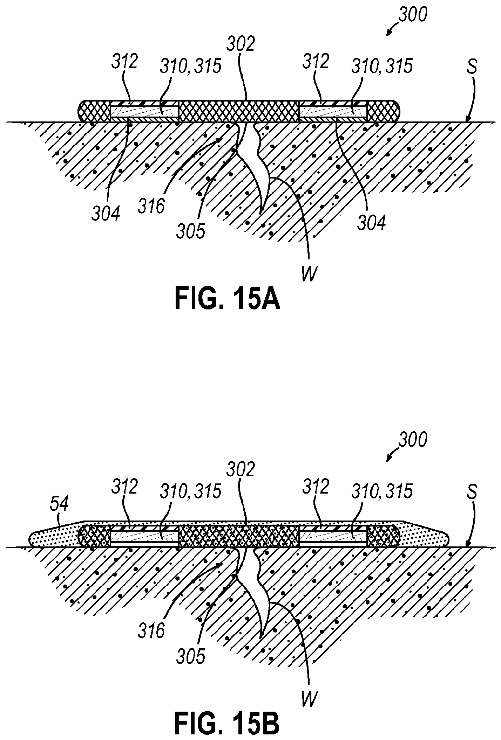

FIGS. 15A-15B show an alternative wound closure device (300) that is substantially similar to wound closure device (120, 120', 140, 160, 180, 200, 220, 240, 260, 280) described above, with differences elaborated herein. Therefore, wound closure device (300) may be readily incorporated into wound closure system (10) in replacement of wound closure device (20, 120, 120', 140, 160, 180, 200, 220, 240, 260, 280) described above. Wound closure device (300) is configured to inhibit topical skin adhesive (54) from curing between an underside (305) of mesh (302) and intact skin (S) in order to promote suitable diffusion/migration of sensate onto intact skin (S). Wound closure device (300) includes a textile mesh (302), pressure sensitive adhesive (304), an underside (305), and sensate depot (310); which may be substantially similar to textile mesh (122, 142, 162, 182, 202, 222, 242, 262, 282), pressure sensitive adhesive (124, 144, 244, 264, 284), underside (125, 145, 165, 185, 205, 225, 245, 265, 285), and sensate depot (130, 150, 170, 190, 210, 230, 250, 270) described above, with differences elaborated herein.

Additionally, wound closure device (300) also includes a protective element in the form of a mask (312). Mask (312) is located adjacent to an upper surface of mesh (302) directly adjacent to sensate depot (310). Mask (312) is formed of a suitable material configured to inhibit uncured topical skin adhesive (54) from penetrating through mask (312) while adhesive (54) is applied in accordance with the description herein. In some instances, mask (312) may be impervious to topical skin adhesive (54); while in other instances, mask (312) may be configured to suitably inhibit topical skin adhesive (54) from reaching underside (305) prior to curing. Therefore, while adhesive spreader (60) pushes topical skin adhesive (54) through portions of mesh (302), mask (312) may act as a sheath surrounding the top surface of wound closure device (300), thereby inhibiting topical skin adhesive (54) from reaching the portion of underside (305) and/or pressure sensitive adhesive (304) directly under sensate depot (310) and skin (S). With mask (312) being located above the top surface of sensate depot (310), mask (312) is configured to inhibit adhesive (54) from reaching and curing at a location interposed between intact skin (S) and underside the sensate depot (310). As such, mask (312) may help ensure sensate (315) suitably migrates/diffuses from sensate depot (310) onto intact skin (S).

Sensate depots (310) are also laterally spaced from the lateral center of mesh (302) directly adjacent to wound (W) such that wound closure device (300) defines an isolated wound area (316). Additionally, the portions of topical skin adhesive (54) that cure on the underside (305) of mesh (302) between wound (W) and sensate depot (310) create a sealed barrier inhibiting sensate (315) from undesirably reaching wound (W). Therefore, mask (312) ensures sensate (315) may be delivered suitable portions of intact skin (S), while cured topical skin adhesive (54) inhibits undesirable migration of sensate towards wound (W).

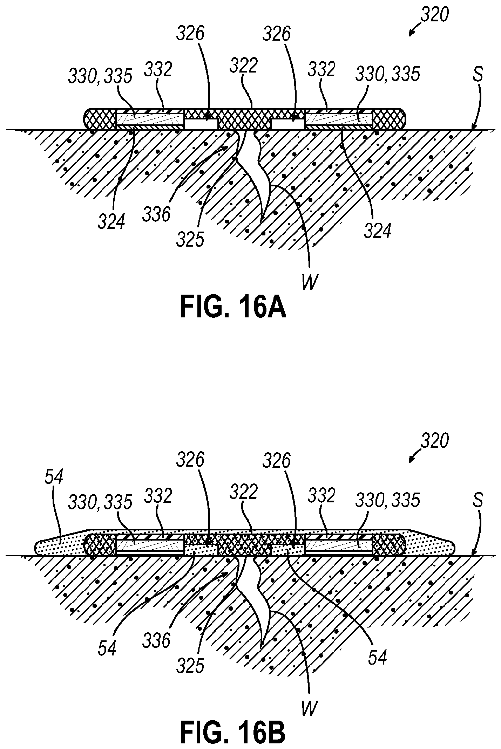

FIGS. 16A-16B show an alternative wound closure device (320) that is substantially similar to wound closure device (300) described above, with differences elaborated herein. Therefore, wound closure device (320) may be readily incorporated into wound closure system (10) in replacement of wound closure device (300) described above. Wound closure device (320) is configured to inhibit topical skin adhesive (54) from curing between an underside (325) of mesh (322) and intact skin (S) in order to promote suitable diffusion/migration of sensate onto intact skin (S). Wound closure device (320) includes a textile mesh (322), pressure sensitive adhesive (324), an underside (325), a sensate depot (330), and a mask (332); which may be substantially similar to textile mesh (302), pressure sensitive adhesive (304), underside (305), sensate depot (310), and mask (312) described above, with differences elaborated herein.

In particular, mesh (322) of wound closure device (320) defines an internal recess (326) extends around isolated wound area (336). In some instances, internal recess (326) may extend continuously to define a perimeter around isolated wound area (336). In other instances, internal recess (326) may extend as an array of recesses (326) to define isolated wound areas (336). In other instances, internal recess (326) may extend around isolated wound area (336) and also have terminating ends such that internal recess (326) does not extend in a continuous fashion. Internal recess (326) is dimensioned to house liquid topical skin adhesive (54) in response to adhesive spreader (60) pushing liquid topical skin adhesive (54) through mesh (322) in accordance with the description herein. Therefore, the portions of topical skin adhesive (54) that cure within recess (326) defined by mesh (322) help create and promote a sealed barrier inhibiting sensate (315) from undesirably reaching wound (W).

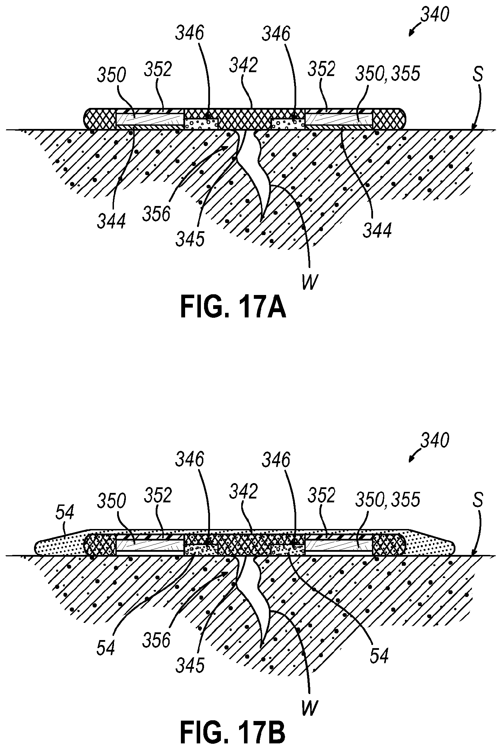

FIGS. 17A-17B show an alternative wound closure device (340) that is substantially similar to wound closure device (320) described above, with differences elaborated herein. Therefore, wound closure device (340) may be readily incorporated into wound closure system (10) in replacement of wound closure device (320) described above. Wound closure device (340) is configured to inhibit topical skin adhesive (54) from curing between an underside (345) of mesh (342) and intact skin (S) in order to promote suitable diffusion/migration of sensate onto intact skin (S). Wound closure device (340) includes a textile mesh (342), pressure sensitive adhesive (344), an underside (345), a sensate depot (350), and a mask (352); which may be substantially similar to textile mesh (322), pressure sensitive adhesive (324), underside (325), sensate depot (330), and mask (332) described above, with differences elaborated herein.

In particular, instead of defining an internal recess, mesh (342) of wound closure device (340) includes an absorbent material (346) extends around isolated wound area (356). In some instances, absorbent material (346) may extend continuously to define a perimeter around isolated wound area (356). In other instances, absorbent material (346) may extend as an array of bodies to define isolated wound areas (356). In other instances, absorbent material (346) may extend around isolated wound area (356) and also have terminating ends such that absorbent material (346) does not extend in a continuous fashion. Absorbent material (346) is configured to absorb liquid topical skin adhesive (54) in response to adhesive spreader (60) pushing liquid topical skin adhesive (54) through mesh (342) in accordance with the description herein. Therefore, the portions of topical skin adhesive (54) that cure within absorbent material (346) help create and promote a sealed barrier inhibiting sensate (355) from undesirably reaching wound (W).

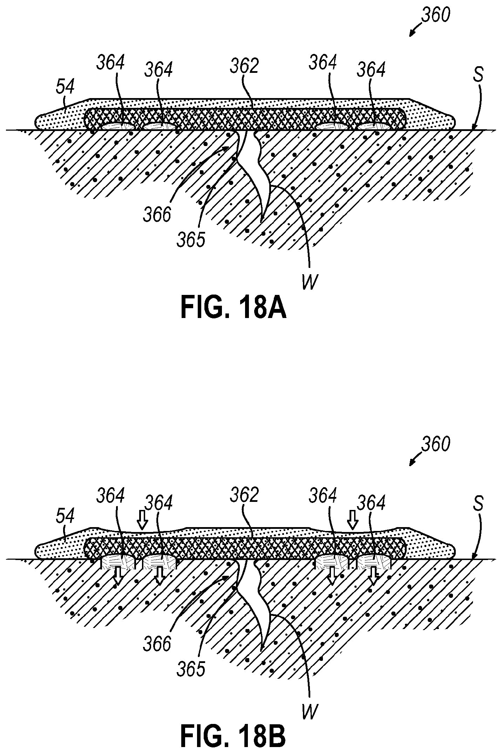

In some instances, it may be desirable to selectively release sensate onto intact skin (S) located within the composite microbial barrier, rather than allow sensate to diffuse/migrate away from a sensate depot. FIGS. 18A-18B show an alternative wound closure device (360) that is substantially similar to wound closure device (120, 120', 140, 160, 180, 200, 220, 240, 260, 280, 300, 320, 340) described above, with differences elaborated herein. Therefore, wound closure device (360) may be readily incorporated into wound closure system (10) in replacement of wound closure device (20, 120, 120', 140, 160, 180, 200, 220, 240, 260, 280, 300, 320, 340) described above.

Therefore, wound closure device (360) includes a textile mesh (362), pressure sensitive adhesive (not shown), and an underside (365); which may be substantially similar to textile mesh (122, 142, 162, 182, 202, 222, 242, 262, 282), pressure sensitive adhesive (124, 144, 244, 264, 284, 304, 324, 344), and underside (125, 145, 165, 185, 205, 225, 245, 265, 285, 305, 325, 345) described above, with differences elaborated herein. However, in the current example, rather than having sensate depots that allow sensate to diffuse over time onto intact skin, wound closure device (360) includes a plurality of rupturable sensate pockets (364) that are laterally spaced from the lateral center of mesh (362) directly adjacent to wound (W) such that wound closure device (360) defines an isolated wound area (366). As shown in FIGS. 18B, if it is desirable to release sensate from rupturable pockets (364), a downward force may be imparted onto mesh (362), thereby rupturing the pocket (364) and releasing sensate. Therefore, sensate may be distributed onto intact skin (S) in a controlled manner as a select time, if desired. It should be understood released sensate may be inhibited from reaching wound (W) via any suitable means as would be apparent to one skilled in the art in view of the teaching herein.

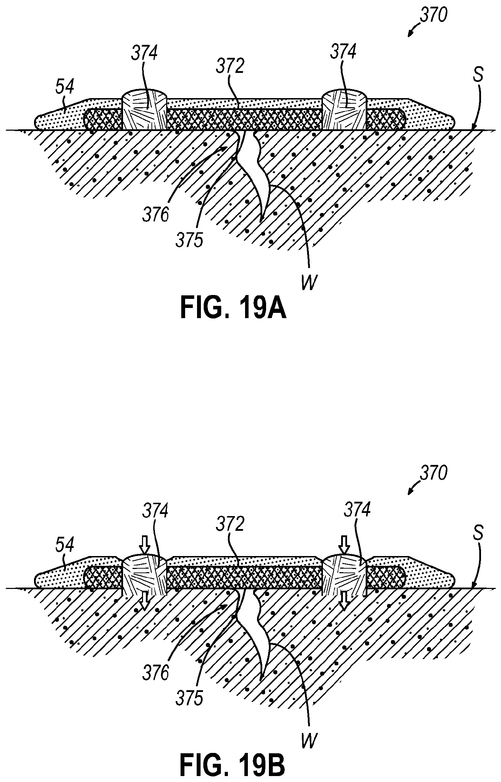

FIGS. 19A-19B show another wound closure device (370) that may be substantially similar to wound closure device (360) described above. Therefore, wound closure device (370) includes a textile mesh (372), pressure sensitive adhesive (not shown), and an underside (375); which may be substantially similar to textile mesh (362), pressure sensitive adhesive (not shown), and underside (365) described above, with differences elaborated herein. Rather than having rupturable sensate pockets (364) completely housed within the composite microbial barrier, wound closure device (370) includes selectively rupturable blisters (374) filled with sensate. Rupturable blisters (374) are laterally spaced from the lateral center of mesh (372) directly adjacent to wound (W) such that wound closure device (370) defines an isolated wound area (376). Rupturable blisters (374) extend upward from cured topical skin adhesive (54) such that individual blisters (374) may be ruptured to release sensate at targeted locations. As shown in FIGS. 19B, if it is desirable to release sensate from rupturable blister (374), a downward force may be imparted onto blister (374) itself, thereby rupturing individual blister (374) and releasing sensate. Therefore, sensate may be distributed onto intact skin (S) in a controlled manner as a select time, if desired. It should be understood released sensate may be inhibited from reaching wound (W) via any suitable means as would be apparent to one skilled in the art in view of the teaching herein.

### V. Miscellaneous

It is understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Furthermore, any one or more of the teachings herein may be combined with any one or more of the teachings disclosed in U.S. Pat. Pub. No. US20250065010A1, entitled "Low Temperature Curable Liquid Silicone Rubber Carrier for Active Pharmaceutical Ingredient," filed on even date herewith; and/or U.S. Pat. Pub. No. US2025065021A1, entitled "Wound Closure System Having Medicant," filed on even date herewith.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DA VINCI^{®} system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK^{®} bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A skin closure system, comprising:
(a) a surgical mesh comprising:
(i) an upper side,
(ii) a lower side,
(iii) a central area, and
(iv) a peripheral area surrounding the central area,
wherein the lower side is configured to contact a section of skin of a patient that surrounds a wound such that the central area is positioned over the wound and the peripheral area is positioned around the wound yet not over the wound;
(b) a topical skin adhesive configured to be applied to the upper side of the surgical mesh, wherein the topical skin adhesive is configured to cure within the surgical mesh to thereby form a protective layer over the wound; and
(c) a sensate located on the peripheral area of the surgical mesh such that the sensate is spaced a distance from the wound.

2. The skin closure system of claim 1, wherein the sensate is configured to contact a portion of skin directly under the peripheral area of the surgical mesh.

3. The skin closure system of any one of claims 1-2, wherein the sensate is configured to be isolated from the wound after the protective layer is formed over the wound.

4. The skin closure system of any one of claims 1-3, wherein the topical skin adhesive, after curing, is configured to form an isolated area around the wound thereby preventing migration of the sensate into the wound.

5. The skin closure system of any one of claims 1-4, wherein the lower side of the surgical mesh defines a recessed layer extending at least partially around the central area between the sensate and the central area, wherein the recess is configured to receive the topical skin adhesive that forms the isolated area.

6. The skin closure system of any one of claims 1-5, wherein the surgical mesh further comprises an absorbent strip extending at least partially around the central area between the sensate and the central area, wherein the absorbent strip is configured to receive the topical skin adhesive that forms the isolated area.

7. The skin closure system of any one of claims 1-6, further comprising a sensate depot associated with the peripheral area of the surgical mesh, wherein the sensate is incorporated into the sensate depot.

8. The skin closure system of claim 7, wherein the sensate depot comprises a material configured to inhibit the topical skin adhesive from penetrating into and underneath the sensate depot.

9. The skin closure system of claim 7, further comprising a mask positioned over the sensate depot, wherein the mask is configured to inhibit the topical skin adhesive from penetrating underneath the sensate depot.

10. The skin closure system of any one of claims 1-9, further comprising a pressure sensitive adhesive located on the lower side of the surgical mesh, wherein the pressure sensitive adhesive is configured to prevent the topical skin adhesive from curing between the sensate and a portion of the skin directly under the sensate.

11. The skin closure system of claim 10, wherein the sensate is incorporated into the pressure sensitive adhesive.

12. The skin closure system of claim 10, wherein the sensate is incorporated into a sensate depot associated with the peripheral area of the surgical mesh, wherein the pressure sensitive adhesive is located underneath the sensate depot.

13. The skin closure system of any one of claims 10-12, wherein the pressure sensitive adhesive comprises a soluble material.

14. The skin closure system of any one of claims 1-13, wherein the sensate comprises at least one of the following therapeutic agents: capsaicin, capsicum, lidocaine, menthol, methyl salicylate, glycol salicylate, topical nonsteroidal anti-inflammatory agents, or camphor.

15. The skin closure system of any one of claims 1-14, wherein the sensate is housed in a rupturable capsule associated with the surgical mesh.

## Patentansprüche

1. Hautverschlusssystem, umfassend:
(a) ein chirurgisches Netz, umfassend:
(i) eine Oberseite,
(ii) eine Unterseite,
(iii) einen zentralen Bereich, und
(iv) einen den zentralen Bereich umgebenden Umfangsbereich,
wobei die Unterseite konfiguriert ist, um eine Hautpartie eines Patienten zu berühren, die eine Wunde derart umgibt, dass der zentrale Bereich über der Wunde positioniert ist und der Umfangsbereich um die Wunde herum, jedoch nicht über der Wunde positioniert ist;
(b) einen topischen Hautklebstoff, der konfiguriert ist, um auf die Oberseite des chirurgischen Netzes aufgebracht zu werden, wobei der topische Hautklebstoff konfiguriert ist, um innerhalb des chirurgischen Netzes auszuhärten, um dadurch eine Schutzschicht über der Wunde auszubilden; und
(c) ein Sensat, das sich in dem Umfangsbereich des chirurgischen Netzes derart befindet, dass das Sensat um eine Entfernung von der Wunde beabstandet ist.

2. Hautverschlusssystem nach Anspruch 1, wobei das Sensat konfiguriert ist, um einen Hautabschnitt direkt unter dem Umfangsbereich des chirurgischen Netzes zu berühren.

3. Hautverschlusssystem nach einem der Ansprüche 1 bis 2, wobei das Sensat konfiguriert ist, um von der Wunde isoliert zu werden, nachdem die Schutzschicht über der Wunde ausgebildet wurde.

4. Hautverschlusssystem nach einem der Ansprüche 1 bis 3, wobei der topische Hautklebstoff nach dem Aushärten konfiguriert ist, um einen isolierten Bereich um die Wunde herum auszubilden, wodurch eine Migration des Sensats in die Wunde verhindert wird.

5. Hautverschlusssystem nach einem der Ansprüche 1 bis 4, wobei die Unterseite des chirurgischen Netzes eine vertiefte Schicht definiert, die sich mindestens teilweise um den zentralen Bereich zwischen dem Sensat und dem zentralen Bereich herum erstreckt, wobei die Vertiefung konfiguriert ist, um den topischen Hautklebstoff aufzunehmen, der den isolierten Bereich ausbildet.

6. Hautverschlusssystem nach einem der Ansprüche 1 bis 5, wobei das chirurgische Netz ferner einen absorbierenden Streifen umfasst, der sich mindestens teilweise um den zentralen Bereich zwischen dem Sensat und dem zentralen Bereich herum erstreckt, wobei der absorbierende Streifen konfiguriert ist, um den topischen Hautklebstoff aufzunehmen, der den isolierten Bereich ausbildet.

7. Hautverschlusssystem nach einem der Ansprüche 1 bis 6, ferner umfassend ein Sensatdepot, das dem Umfangsbereich des chirurgischen Netzes zugeordnet ist, wobei das Sensat in das Sensatdepot eingearbeitet ist.

8. Hautverschlusssystem nach Anspruch 7, wobei das Sensatdepot ein Material umfasst, das konfiguriert ist, um ein Eindringen des topischen Hautklebstoffs in und unter das Sensatdepot zu unterbinden.

9. Hautverschlusssystem nach Anspruch 7, ferner umfassend eine über dem sensorischen Depot positionierte Maske, wobei die Maske konfiguriert ist, um das Eindringen des topischen Hautklebstoffs unter das Sensatdepot zu unterbinden.

10. Hautverschlusssystem nach einem der Ansprüche 1 bis 9, ferner umfassend einen druckempfindlichen Klebstoff, der sich auf der Unterseite des chirurgischen Netzes befindet, wobei der druckempfindliche Klebstoff konfiguriert ist, um das Aushärten des topischen Hautklebstoffs zwischen dem Sensat und einem Abschnitt der Haut direkt unter dem Sensat zu verhindern.

11. Hautverschlusssystem nach Anspruch 10, wobei das Sensat in den Haftklebstoff eingearbeitet ist.

12. Hautverschlusssystem nach Anspruch 10, wobei das Sensat in ein Sensatdepot eingearbeitet ist, das dem Umfangsbereich des chirurgischen Netzes zugeordnet ist, wobei sich der Haftklebstoff unter dem Sensatdepot befindet.

13. Hautverschlusssystem nach einem der Ansprüche 10 bis 12, wobei der druckempfindliche Klebstoff ein lösliches Material umfasst.

14. Hautverschlusssystem nach einem der Ansprüche 1 bis 13, wobei das Sensat mindestens eines der folgenden therapeutischen Mittel umfasst:
Capsaicin, Capsicum, Lidocain, Menthol, Methylsalicylat, Glycolsalicylat, topische nichtsteroidale entzündungshemmende Mittel oder Campher.

15. Hautverschlusssystem nach einem der Ansprüche 1 bis 14, wobei das Sensat in einer zerreißbaren Kapsel untergebracht ist, die dem chirurgischen Netz zugeordnet ist.

## Revendications

1. Système de fermeture de peau comprenant :
(a) un filet chirurgical comprenant :
(i) un côté supérieur,
(ii) un côté inférieur,
(iii) une zone centrale, et
(iv) une zone périphérique entourant la zone centrale,
dans lequel le côté inférieur est conçu pour venir en contact avec une section de peau d'un patient qui entoure une plaie de telle sorte que la zone centrale est positionnée par-dessus la plaie et la zone périphérique est positionnée autour de la plaie, mais pas par-dessus la plaie ;
(b) un adhésif topique pour la peau conçu pour être appliqué au côté supérieur du filet chirurgical, dans lequel l'adhésif topique pour la peau est conçu pour durcir au sein du filet chirurgical pour former de ce fait une couche protectrice par-dessus la plaie ; et
(c) un agent sensoriel localisé sur la zone périphérique du filet chirurgical de telle sorte que l'agent sensoriel est espacé à une certaine distance de la plaie.

2. Système de fermeture de peau selon la revendication 1, dans lequel l'agent sensoriel est conçu pour venir en contact avec une partie de peau directement sous la zone périphérique du filet chirurgical.

3. Système de fermeture de peau selon l'une quelconque des revendications 1 à 2, dans lequel l'agent sensoriel est conçu pour être isolé de la plaie après que la couche protectrice a été formée par-dessus la plaie.

4. Système de fermeture de peau selon l'une quelconque des revendications 1 à 3, dans lequel l'adhésif topique pour la peau, après durcissement, est conçu pour former une zone isolée autour de la plaie empêchant de ce fait une migration de l'agent sensoriel dans la plaie.

5. Système de fermeture de peau selon l'une quelconque des revendications 1 à 4, dans lequel le côté inférieur du filet chirurgical définit une couche en retrait s'étendant au moins partiellement autour de la zone centrale entre l'agent sensoriel et la zone centrale, dans lequel le retrait est conçu pour recevoir l'adhésif topique pour la peau qui forme la zone isolée.

6. Système de fermeture de peau selon l'une quelconque des revendications 1 à 5, dans lequel le filet chirurgical comprend en outre une bande absorbante s'étendant au moins partiellement autour de la zone centrale entre l'agent sensoriel et la zone centrale, dans lequel la bande absorbante est conçue pour recevoir l'adhésif topique pour la peau qui forme la zone isolée.

7. Système de fermeture de peau selon l'une quelconque des revendications 1 à 6, comprenant en outre un dépôt d'agent sensoriel associé à la zone périphérique du filet chirurgical, dans lequel l'agent sensoriel est incorporé dans le dépôt d'agent sensoriel.

8. Système de fermeture de peau selon la revendication 7, dans lequel le dépôt d'agent sensoriel comprend un matériau conçu pour empêcher l'adhésif topique pour la peau de pénétrer dans le, et en dessous du, dépôt d'agent sensoriel.

9. Système de fermeture de peau selon la revendication 7, comprenant en outre un masque positionné par-dessus le dépôt d'agent sensoriel, dans lequel le masque est conçu pour empêcher l'adhésif topique pour la peau de pénétrer en dessous du dépôt d'agent sensoriel.

10. Système de fermeture de peau selon l'une quelconque des revendications 1 à 9, comprenant en outre un adhésif sensible à la pression localisé sur le côté inférieur du filet chirurgical, dans lequel l'adhésif sensible à la pression est conçu pour empêcher l'adhésif topique pour la peau de durcir entre l'agent sensoriel et une partie de la peau directement sous l'agent sensoriel.

11. Système de fermeture de peau selon la revendication 10, dans lequel l'agent sensoriel est incorporé dans l'adhésif sensible à la pression.

12. Système de fermeture de peau selon la revendication 10, dans lequel l'agent sensoriel est incorporé dans un dépôt d'agent sensoriel associé à la zone périphérique du filet chirurgical, dans lequel l'adhésif sensible à la pression est localisé en dessous du dépôt d'agent sensoriel.

13. Système de fermeture de peau selon l'une quelconque des revendications 10 à 12, dans lequel l'adhésif sensible à la pression comprend un matériau soluble.

14. Système de fermeture de peau selon l'une quelconque des revendications 1 à 13, dans lequel l'agent sensoriel comprend au moins l'un des agents thérapeutiques suivants : capsaïcine, capsicum, lidocaïne, menthol, salicylate de méthyle, salicylate de glycol, agents anti-inflammatoires non stéroïdiens topiques, ou camphre.

15. Système de fermeture de peau selon l'une quelconque des revendications 1 à 14, dans lequel l'agent sensoriel est logé dans une capsule frangible associée au filet chirurgical.
